(19) Europäisches Patentamt / European Patent Office / Office européen des brevets

(11) **EP 2 972 311 B1**

(12) **EUROPEAN PATENT SPECIFICATION**

(45) Date of publication and mention of the grant of the patent:
**11.11.2020 Bulletin 2020/46**

(51) Int Cl.:
***G16B 15/30*** *(2019.01)*

(21) Application number: **14769374.1**

(22) Date of filing: **13.03.2014**

(86) International application number:
**PCT/US2014/025425**

(87) International publication number:
**WO 2014/151310 (25.09.2014 Gazette 2014/39)**

(54) **CYCLE CLOSURE ESTIMATION OF RELATIVE BINDING AFFINITIES AND ERRORS**

SCHÄTZUNG DER ZYKLUSSCHLIESSUNG VON RELATIVEN BINDUNGSAFFINITÄTEN UND FEHLERN

ESTIMATION DE FERMETURES DE CYCLES DANS LES AFFINITÉS ET LES ERREURS DE LIAISON RELATIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201313840039**

(43) Date of publication of application:
**20.01.2016 Bulletin 2016/03**

(73) Proprietor: **Schrodinger, LLC**
**New York, New York 10036-4041 (US)**

(72) Inventors:
• **WANG, Lingle**
  **New York NY 10044-0089 (US)**
• **LIN, Teng**
  **Manalapan**
  **New Jersey 07726 (US)**
• **ABEL, Robert**
  **Brooklyn**
  **New York 11201 (US)**

(74) Representative: **Fish & Richardson P.C.**
**Highlight Business Towers**
**Mies-van-der-Rohe-Straße 8**
**80807 München (DE)**

(56) References cited:
WO-A1-2012/168697     WO-A2-2012/109383
US-A1- 2003 176 656     US-A1- 2010 241 411
US-A1- 2012 258 518     US-A1- 2012 303 292

• L. WANG ET AL: "On achieving high accuracy and reliability in the calculation of relative protein-ligand binding affinities", PROCEEDINGS OF THE NATIONAL ACADEMY OF SCIENCES, vol. 109, no. 6, 7 February 2012 (2012-02-07), pages 1937-1942, XP055312031, US ISSN: 0027-8424, DOI: 10.1073/pnas.1114017109

• LINGLE WANG ET AL: "Replica Exchange with Solute Scaling: A More Efficient Version of Replica Exchange with Solute Tempering (REST2)", JOURNAL OF PHYSICAL CHEMISTRY PART B: CONDENSED MATTER, MATERIALS, SURFACES, INTERFACES & BIOPHYSICAL, vol. 115, no. 30, 4 August 2011 (2011-08-04), pages 9431-9438, XP055312153, US ISSN: 1520-6106, DOI: 10.1021/jp204407d

• D L BEVERIDGE ET AL: "Free Energy Via Molecular Simulation: Applications to Chemical and Biomolecular Systems", ANNUAL REVIEW OF BIOPHYSICS AND BIOENGINEERING., vol. 18, no. 1, 1 June 1989 (1989-06-01), pages 431-492, XP055312294, US ISSN: 0883-9182, DOI: 10.1146/annurev.bb.18.060189.002243

• COZZINI ET AL.: 'Free Energy of Ligand Binding to Protein: Evaluation of the Contribution of Water Molecules by Computational Methods.' CURRENT MEDICINAL CHEMISTRY December 2004, XP002459639 Retrieved from the Internet: <URL:http://www.ncbi.nlm.nih.gov/pubmed/15579003> [retrieved on 2014-08-22]

- Andrew Pohorille ET AL: "A Bayesian Approach to Calculating Free Energies in Chemical and Biological Systems", AIP CONFERENCE PROCEEDINGS, vol. 872, 1 January 2006 (2006-01-01), pages 23-30, XP055692870, NEW YORK, US ISSN: 0094-243X, DOI: 10.1063/1.2423257

**Description**

**CLAIM OF PRIORITY**

This application claims priority to U.S. Patent Application Serial No. 13/840,039, filed on March 15, 2013.

**TECHNICAL FIELD**

This invention is in the general field of bioinformatics, more specifically estimating or calculating relative receptor/ligand binding affinities and their errors.

**BACKGROUND**

Biological processes often depend on protein-ligand binding events (for example, binding of a ligand to its receptor), and thus accurate calculation of the associated energetics is a central goal of computational structure-based drug design.[1-3]

A variety of different approaches have been developed to calculate protein-ligand binding free energies with different trade-offs between speed and accuracy, including the fast end point methods such as empirical scoring functions in virtual screening and molecular mechanics/Generalized Born surface area (MM/GBSA) or Poisson-Boltzmann (MM/PBSA) models, the WM/MM method that more explicitly considers the contribution from the desolvation of protein than GB methods, and free energy perturbation (FEP) and thermodynamic integration (TI) methods designed to consider a complete or essentially complete energetic description of binding within the accuracy limits of the underlying force field and complete sampling.[1,2,4-10]

Among the various methods to calculate protein-ligand binding affinities, free energy calculations-such as TI, FEP, lambda dynamics, alchemical OSRW, etc-generally provide a thermodynamically complete description of the binding event and, at least in theory, yield accurate predictions within the limits of the force field used and complete sampling of the phase space. However, practical application of free energy calculations in industrial contexts may be limited by the large computational resources required, particularly when sampling of multiple conformations, the timescales needed to impact live projects, and the need for a robust and meaningful approach to determine the sampling error associated with such free energy calculations. [4, 11, 12]

US 2010/0241411 describes a technique to exhaustively enumerate the thermodynamic properties of the water molecules solvating the active site of a protein in its apostate and calculate the relative binding affinities of congeneric compounds that bind to this protein.

Wang et al. (PNAS 109:6) describes the application of free energy perturbation and free energy perturbation/replica exchange with solute tempering to two modifications of protein-ligand complexes that lead to significant conformational changes.

Cozzini et al. (Curr. Med. Chem. 11:10) describes various methods of evaluating the contribution of water molecules to the free energy of ligand binding to proteins.

Beveridge et al., Ann. Rev. Biophys. Bioeng. 18:1) reviews free energy calculations via molecular simulations.

**SUMMARY**

We have discovered a generalizable computer-implemented method for improving free energy calculations of relative receptor-ligand binding affinities, particularly for estimating the relative binding affinities of congeneric sets of ligands and determining the sampling errors associated with those estimates.

One aspect of the invention generally features a computer-implemented method of determining relative strength of binding between a receptor and individual members of a set ligands to form complexes between individual ligand set members and the receptor. The method includes a computer-implemented determination of multiple relative binding free energy differences for a set of ligand pairs forming at least one closed cycle, and a computer implemented determination of hysteresis magnitude about each closed thermodynamic cycle. In general, the method includes,

    a. a computer implemented probabilistic determination of the free energy differences and error distributions about those free energy differences along each of the legs of the closed thermodynamic cycle that probabilistically lead to the hysteresis value observed for the closed thermodynamic cycles;

    b. a computer implemented determination of the most probable free energy difference for each leg in the closed thermodynamic cycle included in the probabilistic model determined in a;

    c. a computer implemented determination of the most probable error associated with the most probable binding free energy difference for each pair of ligands along each leg in the closed thermodynamic cycle from the probabilistic determination in b.;
d. a computer implemented output of values representing the relative binding free energies and errors in c. to a computer user.

In preferred embodiments, the step of estimating the error comprises computer-implemented analysis of binding free energy differences between ligands along legs of more than one closed thermodynamic cycle, and computer implemented determination of hysteresis magnitude about each of closed thermodynamic cycles. Also in preferred embodiements, steps c and d include determining a set of free energy values for each leg that minimizes the function

$$\sum_i \frac{(E_i - F_i)^2}{2\sigma_i^2}$$

with the constraint:

$$\sum_i F_i = 0$$

where Ei calculated free energy difference for a given leg i;
$F_i$ is the theoretical free energy difference for a given leg i;
and $\sigma_i$ is the standard deviation of the calculated free energy differences for leg i, and where the sum of the theoretical free energy differences for all closed cycles is 0.

In other preferred embodiments: the receptor is a protein; the ligands are congeneric; a Gaussian distribution is assumed in the construction of the probabilistic model of the observed hysteresis in step a.; the error distribution associated with the free energy simulations in step a. is assumed to be uniform; the error distribution is assumed to be additive with the Bennett error in step a. The connectivity of the closed thermodynamic cycles is represented as a graph or as a matrix. The probablisitic determination can be done by various methods, including without limitation, graph theoretical methods, matrix algebra methods, Bayesian methods, Maximum likelihood methods.

Other aspects of the invention feature a computer readable medium comprising tangible non-transitory instructions for performing the above-described methods, and a general purpose graphics processing unit with non-transitory computer readable instructions for performing those methods.

Below, we exemplify the invention of the cycle closure affinity estimation and error analysis machinery to FEP/REST (Free Energy Perturbation/Replica Exchange with Solute Tempering),[12-15] to FEP/MD, and to TI relative binding affinity free energy calculations, but the invention can be used with a number of other relative binding free energy calculation platforms, including lambda dynamics, alchemical metadynamics, alchemical OSRW, or others. The invention can also be employed irrespective of whether or not the particular relative free energy calculation platform is implemented employing sampling algorithms based on molecular dynamics, monte carlo, grand-canonical monte carlo, replica-exchange molecular dynamics, accelerated molecular dynamics, or any other sampling protocol.

In particular, we exemplify the invention by investigating a subset from a series of congeneric ligands binding to the cyclin dependent kinase CDK2-cyclin A receptor.[16] CDK2 is a member of the CDK family performing various functions in the regulation of the cell proliferation and the RNA polymerase II transcription cycles. CDK2 has also been identified as an important drug target for tumor-selective therapeutic strategies.[17,18]

Notwithstanding the specific systems we present to exemplify the invention, those skilled in the art will understand that the invention can be used as a general tool for ligand binding investigation. To illustrate this point, we have also applied the invention to compute relative binding affinities of ligands binding to JNK kinase and BACE, which are also protein targets of significant medicinally interest.

The details of one or more embodiments of the invention are set forth in the accompanying drawings and in the description below. Other features, objects, and advantages of the invention will be apparent from the description and drawings, and from the claims.

## DESCRIPTION OF THE DRAWINGS

The patent or application file contains at least one drawing executed in color. Copies of this patent or application publication with color drawing(s) will be provided by the Office upon request and payment of the necessary fee.

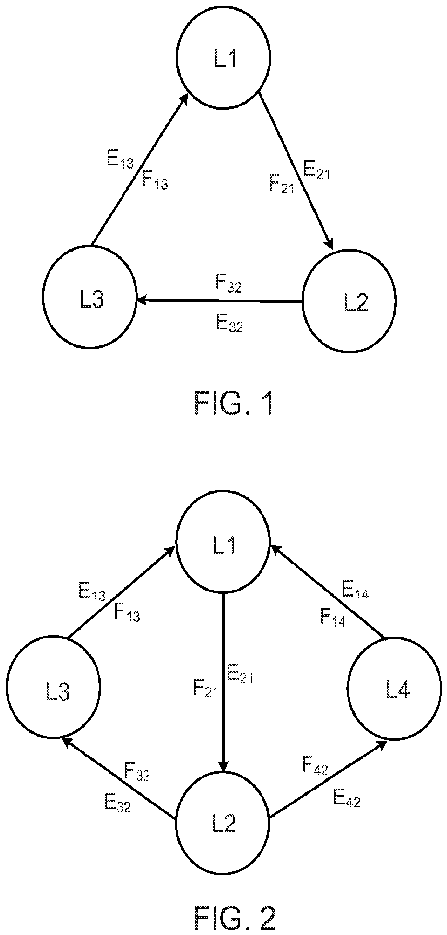

**Figure 1:** A simple model illustrating the problem of consistency and reliability checking in FEP calculations. For a set of three ligands L1, L2, and L3, three FEP calculations were performed with estimated free energy differences E21,E32 and E13 respectively. How to get the best estimates of the relative free energies (F21, F32, and F13) from the three FEP calculations, and how to judge whether the calculations are reliable?

**Figure 2:** A more complicated path with two cycles sharing the same edge used in the CDK2 inhibitors binding affinity calculation.

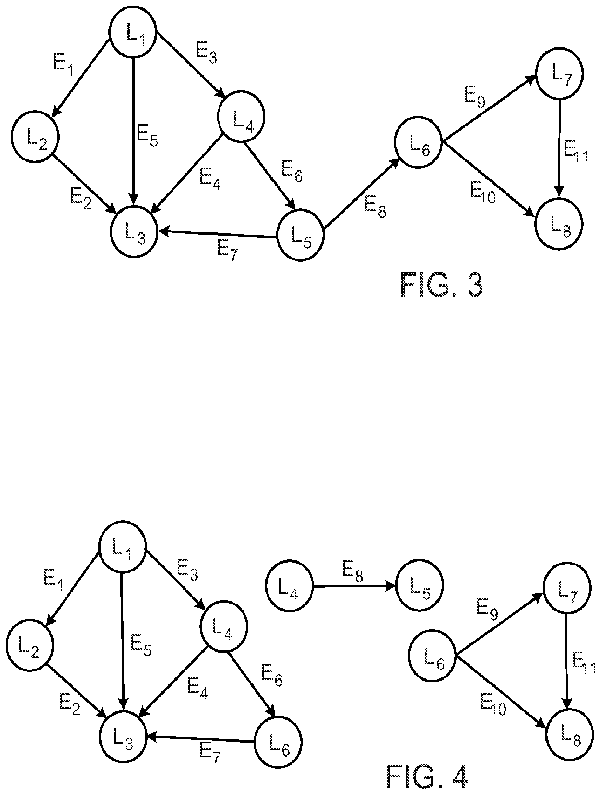

**Figure** 3: Example graph for relative free energy calculations in general applications. Each vertex on the graph represents a ligand. Each edge on the graph represents a relative free energy calculations, the arrow of the edge indicates the direction of the relative binding free energy calculation, and the value on each edge is the calculated free energy difference from the free energy calculation for that particular pair of ligands.

**Figure 4:** Decomposition of the whole graph in Figure 3 into 3 independent subgraphs. The subgraphs may share the same vertex, but they don't share the same edge.

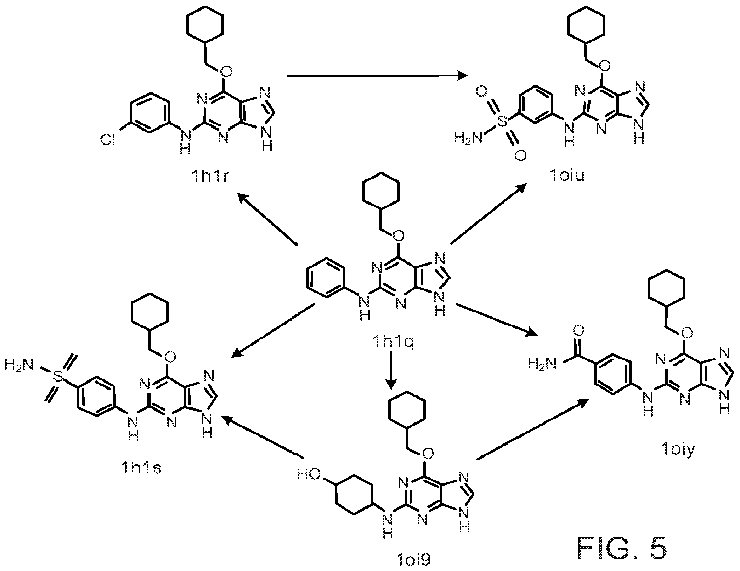

**Figure 5:** The structures of the 6 CDK2 ligands with crystal structures and the paths used for the FEP/REST and FEP/MD calculations. The three red arrows represent the relative binding free energy calculation paths which complete three distinct cycles for assessing the consistency and reliability of the calculations. Ligand 1h1q was used for the 5 blue relative binding free energy calculations since the other compounds can all be generated by simply adding different substituents on the benzene ring of ligand 1h1q.

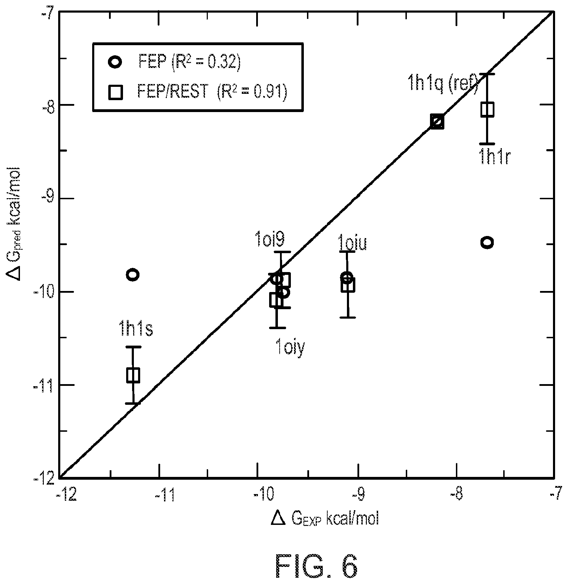

**Figure 6:** The FEP/MD and FEP/REST predicted results versus the experimental binding affinity data for the set of CDK2 inhibitors with crystal structures. The FEP/MD predicted binding affinities do not vary significantly, making it impossible to reliably rank ligands 1h1r, 1h1s, 1oiu, 1oi9, and 1oiy using these values. By contrast, the FEP/REST predicted results have a very high correlation with experimental data (R2=0.91), and the deviation is less than 0.8 kcal/mol for all pairs. The error bars for the FEP/REST predictions reported in this figure are calculated by way of the embodiments of the invention using cycle closure analysis.

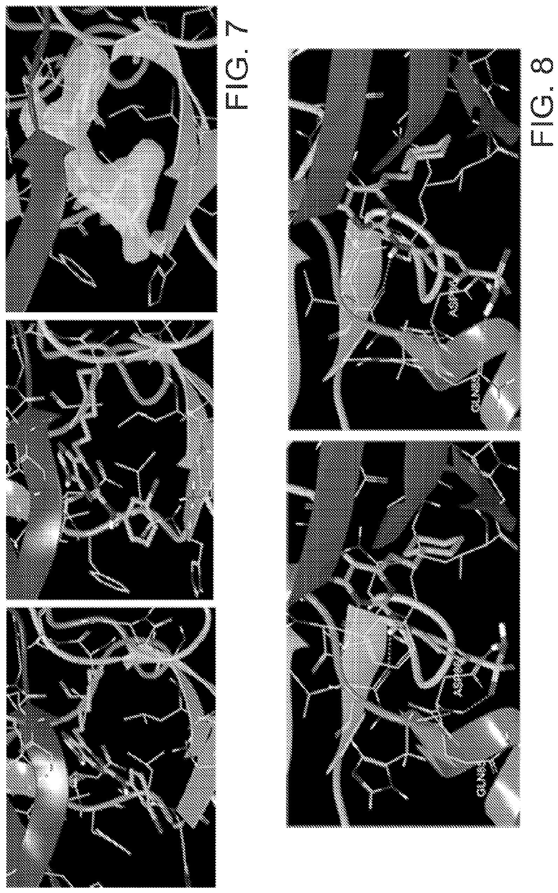

**Figure 7:** The starting conformation corresponding to the crystal structure for the FEP/REST simulation for ligand 1h1r (left), the alternative binding mode, with the chloro-substituted phenyl ring flipped, observed in the REST/REST trajectory (middle), and the electron density of ligand 1h1r in the X-ray crystallography.(right) The proteins are represented by colored ribbons for the backbone and stick representation for the side-chains, while the ligands are depicted in tube representation, and the electron density is displayed as solid yellow surface. The sigma cutoff used for density visualization is 1.0. For ligand 1h1r, the electron density has two peaks for the chloro-substituted benzene ring, indicating that the ligand has two binding modes, consistent with the FEP/REST simulations.

**Figure 8:** The starting conformation corresponding to the crystal structure for FEP/REST simulation for ligand 1h1s (left), and the alternative binding mode observed in the REST/REST trajectory (right). The proteins are represented by colored ribbons for the backbone and stick representation for the side-chains, while the ligands are depicted in tube representation. Compared to the starting conformation, the sulfonamide group rotates about 120° around the C-S bond, and the residues surrounding the binding pocket, especially Gln85 and Asp86, rearrange accordingly. Two hydrogen bonds are formed between the sulfonamide group and the protein residues.

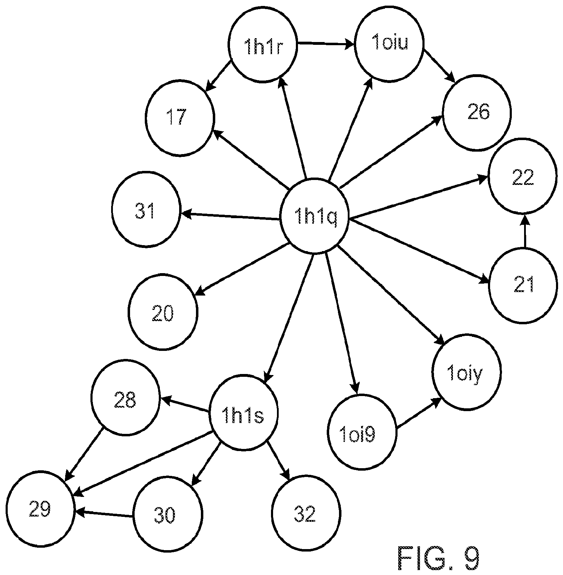

**Figure 9:** The relative binding free energy calculation paths used for the FEP/REST calculations for those ligand without crystal structure binding to CDK2 receptor.

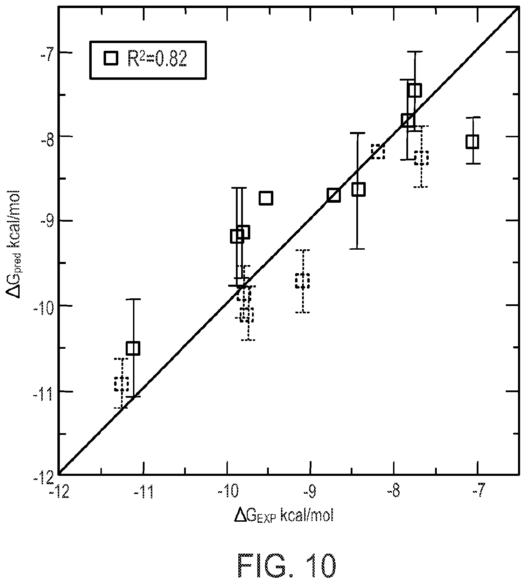

**Figure 10:** The FEP/REST predicted results versus the experimental binding affinity data for the whole set of CDK2 inhibitors including both the 6 ligands with crystal structures and the 10 ligands without crystal structures. The 6 ligands with crystal structures (displayed in Figure 6) are colored red in the figure and the 10 ligands without crystal structures are colored black. The error bars for the FEP/REST predictions reported in this figure are calculated based on the method detailed in Appendix A using cycle closures.

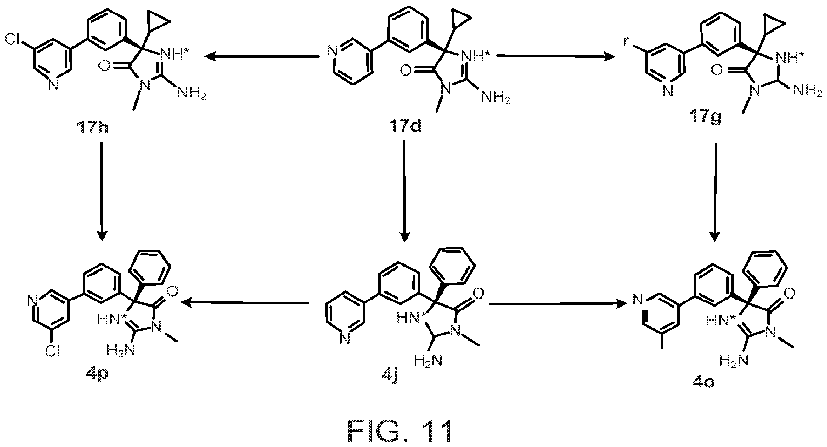

**Figure 11:** Relative biding free energy calculation paths for the 7 ligands binding to Jnk1 receptors with 2 independent cycle closures.

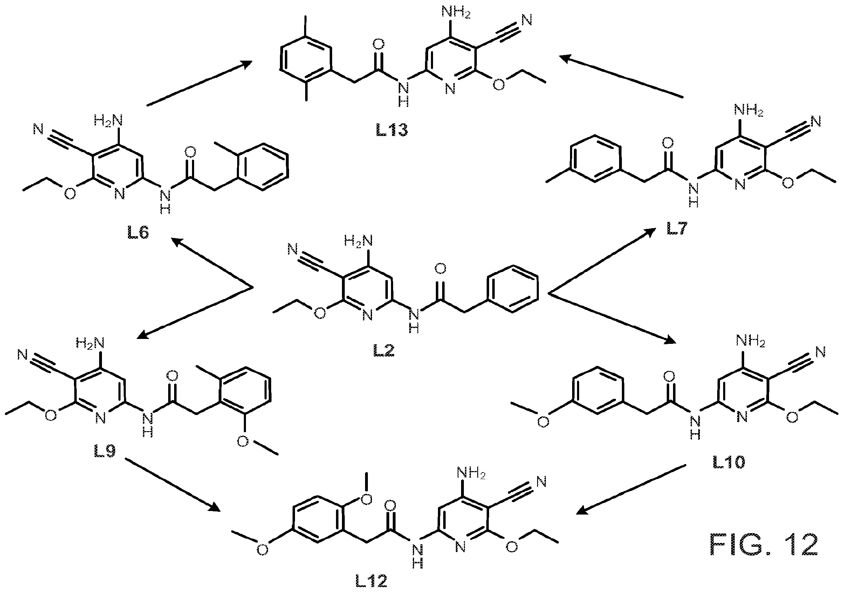

**Figure 12:** Relative binding free energy calculation paths for the 6 ligands binding to BACE with two independent cycle closures.

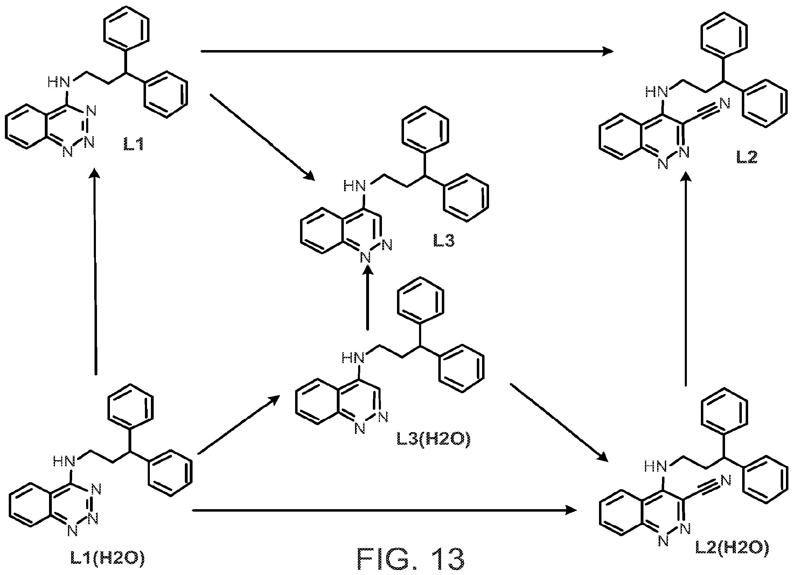

**Figure 13:** Relative binding free energy calculation paths for the 3 ligands binding to scytalone dehydratase with and without water bound in the active site. The simulations with water bound in the active site are indicated by '(H2O)'. 4 independent cycle closures are constructed in the relative binding free energy calculation paths.

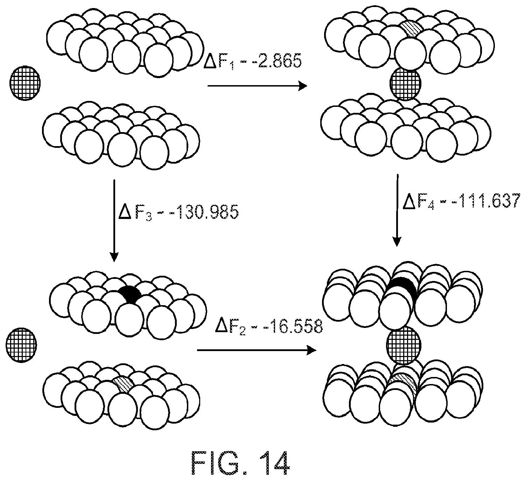

**Figure 14:** Thermodynamic cycles connecting methane-plate binding affinities and the free energies of charging the plates in water. The gray particles represent the Lennard Jones atoms forming the model hydrophobic enclosure, the red particles represent negatively charged ions, blue particles represent positively charged ions, and green particles represent united-atom methane, which binds to the enclosures.

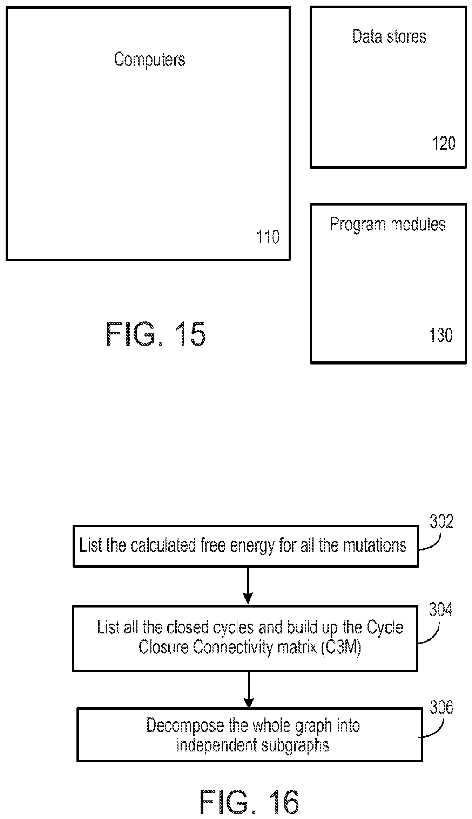

**Figure 15:** A block diagram of a computer system that performs the method.

**Figure 16:** A flow diagram of steps in the method.

## DETAILED DESCRIPTION

Relative binding affinities calculated with the invention using Cycle Closure are highly correlated with the experimental data, and a meaningful error bar is produced indicating the convergence of the calculations.

This is especially apparent when employing the invention to evaluate FEP/REST free energy calculations, which provide thorough sampling of the different conformations of the ligand in the active site of the receptor. While not being bound to a particular theory, we identified that for two of the ligands there are multiple binding modes and that sampling those mode is important for the correct prediction of the binding affinities. While FEP/REST can sample the important conformations in a relatively short simulation time, the ligands were trapped in the initial conformations using FEP/MD. The method illustrates this feature, indicating a high error associated with the FEP/MD free energy calculations, and a low error with the FEP/REST calculations. Thus, the invention can improve relative free energy calculation techniques by providing a reasonable estimate of the associated errors in the calculation and the reliability of the predictions.[19]

Initially, the method builds up cycle closures in relative free energy calculations as has been done by several other groups,[10,20,21] then from this input, the method determines the consistency and reliability of the relative free energy calculations by assessing how much the sum of the calculated free energy changes for each closed thermodynamic cycle deviates from the theoretical value of 0. In the section that follows, we describe the method in detail, such that one skilled in the art can immediately see how the method yields *reliable* predictions of the relative free energies from the multiple free energy estimates obtained by way of the cycle closure relative free energy calculations, as well as the expected error bounds associated with those calculations, as well as mechanism to flag relative free energy calculations that have systematic errors.

### *Detailed Specification of the Cycle Closure Relative Free Energy Consistency Invention*

### *I. Consistency validation and the best free energy estimator using cycle closure in relative binding free energy calculation paths*

The following simple model illustrates the methods and concepts of the method. Consider a set of three ligands, $L_1$, $L_2$, and $L_3$. Suppose the experimentally measured relative binding free energy differences between the three ligands are $F_{21}^{\mathrm{exp}} = F_2^{\mathrm{exp}} - F_1^{\mathrm{exp}}$, $F_{32}^{\mathrm{exp}} = F_3^{\mathrm{exp}} - F_2^{\mathrm{exp}}$, and $F_{13}^{\mathrm{exp}} = F_1^{\mathrm{exp}} - F_3^{\mathrm{exp}}$ where $F_1^{\mathrm{exp}}$, $F_2^{\mathrm{exp}}$, and $F_3^{\mathrm{exp}}$ are the experimentally measured binding free energies for the ligands $L_1$, $L_2$, and $L_3$, respectively. The free energy is a thermodynamic property, therefore $F_{13}^{\mathrm{exp}} + F_{32}^{\mathrm{exp}} + F_{21}^{\mathrm{exp}} = 0$ (See Figure 1). Suppose three relative binding free energy calculations are performed, from $L_1$ to $L_2$, $L_2$ to $L_3$, and $L_3$ to $L_1$, and the calculated free energy differences for the three relative binding free energy calculation paths are $E_{21}$, $E_{32}$ and $E_{13}$ respectively. If the simulations are perfectly converged and the force field is perfect, then ideally $E_{21} = F_{21}^{\mathrm{exp}}$, $E_{32} = F_{32}^{\mathrm{exp}}$, $E_{13} = F_{13}^{\mathrm{exp}}$, and $E_{13} + E_{21} + E_{32} = 0$. In practice, however, there are errors associated with the calculated relative free energies, and in general, $E_{21} \neq F_{21}^{\mathrm{exp}}$, $E_{32} \neq F_{32}^{\mathrm{exp}}$, $E_{13} \neq F_{13}$, and $E_{13} + E_{32} + E_{21} = \Delta \neq 0$. We call $\Delta$, the hysteresis of the free energy calculation associated with this cycle closure. These errors include the unbiased statistical errors due to the random fluctuations and the biased errors due to the incomplete sampling of the phase space (the protein and/or the ligand are trapped in a local minimal in the conformation space) and the error in the force field. The method assesses the consistency and reliability of these calculations.

The errors in relative free energy calculations compared to experimental values can be separated into two categories: the systematic error coming from the difference of the force field used in simulation compared to the true potential energy surface of the system, and the error coming from the nonconvergence, either due to randomly or systematically incomplete sampling of the phase space, or from the free energy estimator itself, eg TI versus FEP, etc. Let $F_{21}$ denote the theoretical free energy difference between two thermodynamic states (ie from ligand $L_1$ to ligand $L_1$) for the underlying force field from an infinite long unbiased simulation and unbiased relative free energy estimator. If there is no systematic error in the force field, then $F_{21} = F_{21}^{\mathrm{exp}}$. In practical relative free energy calculations, for example, for calculation from $L_1$ to $L_2$, the simulation is run with a finite amount of sampling time and the sampling may have some bias, thus the calculated free energy $E_{21}$ might deviate from its theoretical value $F_{21}$, such that it depends on the initial configuration of the simulation.

Repeating the same relative free energy calculation an infinite number of times starting from different initial configurations and different random seeds for the velocities, the calculated free energies have a distribution. Without loss of generality, for the calculation the relative binding free energy from ligand $L_i$ to ligand $L_j$ with theoretical free energy difference between them $F_{ji}$, $P(E_{ji} \mid F_{ji})$ denotes the distribution of the calculated free energy $E_{ji}$. $P(E_{ji} \mid F_{ji})$ can in principle be any kind of distributions, such as the Gaussian distribution, Lorentz distribution, uniform distribution, delta distribution and so on.

$$P(E_{ji} \mid F_{ji}) = \begin{cases} \delta(E_{ji} - F_{ji}) & \delta \text{ distribution} \\ \dfrac{1}{2a} \text{ when } E_{ji} \in [F_{ji} - a, F_{ji} + a] & \text{uniform distribution} \\ \dfrac{1}{\sqrt{2\pi}\sigma} \exp(-\dfrac{(E_{ji} - F_{ji})^2}{2\sigma^2}) & \text{Gaussian distribution} \\ \dots \text{ other distributions} \end{cases}$$

This method predicts the free energy difference from ligand $L_i$ to ligand $L_j$ and the associated error for the prediction based on the distribution $P(E_{ji} \mid F_{ji})$ with cycle closures constructed in the relative binding free energy calculation paths, ie:

$$P(E_{ji} \mid F_{ji}) \mid_{\text{closed cycles in the paths}} \rightarrow \{\hat{E}_{ji}, e_{ji}\}$$

where $\hat{E}_{ji}$ denotes the predicted free energy difference between ligand $L_i$ to ligand $L_j$, and $e_{ji}$ denotes the error associated with the prediction.

The prediction can be done using many different methods, for example, the maximum likelihood method, which maximizes the probability of the observation; the Bayesian statistics method, which optimizes the parameters based on the calculated free energies and so on.

Here, we exemplify the inversion through one specific example, which uses the Maximum Likelihood method and assuming Gaussian distribution for the calculated free energies. Those skilled in the art will understand that the invention can be used as a general tool for other types of statistical analysis methods and other types of distributions. An example derivation using Bayesian statistics is also given at the end of this section.

Assume that the calculated free energies are Gaussian distributed with average $F_{21}$ (no systematic bias) and standard deviation $\sigma_{21}$. Then the probability density that one single relative free energy calculation gives a value of $E_{21}$ for this path is:

$$\rho_{21}(E_{21} \mid F_{21}) = \frac{1}{\sqrt{2\pi}\sigma_{21}} \exp(-\frac{(E_{21} - F_{21})^2}{2\sigma_{21}^2})$$

Similarly, for paths $L_2$ to $L_3$, and $L_3$ to $L_1$, the probability density that the relative free energy calculations give values of $E_{32}$ and $E_{13}$ are respectively:

$$\rho_{32}(E_{32} \mid F_{32}) = \frac{1}{\sqrt{2\pi}\sigma_{32}} \exp(-\frac{(E_{32} - F_{32})^2}{2\sigma_{32}^2})$$

$$\rho_{13}(E_{13} \mid F_{13}) = \frac{1}{\sqrt{2\pi}\sigma_{13}} \exp(-\frac{(E_{13} - F_{13})^2}{2\sigma_{13}^2})$$

For a given set of theoretical free energy differences $F_{21}$, $F_{32}$, and $F_{13}$, the overall likelihood, $L$, that the three relative free energy calculations give values of $E_{21}$, $E_{32}$, and $E_{13}$ is:

$$L(E_{21}, E_{32}, E_{13} \mid F_{21}, F_{32}, F_{13}) = \frac{1}{(2\pi)^{3/2} \sigma_{21} \sigma_{32} \sigma_{13}} \exp(-\sum_i \frac{(E_i - F_i)^2}{2\sigma_i^2}) \text{ where } i \in (21, 32, 13)$$

According to the maximum likelihood method, the most likely values of $F_{21}$, $F_{32}$ and $F_{13}$ are the set of values that maximize the above likelihood. Taking the log of the above expression, the set of values which maximizes the likelihood is the set of values that minimize the following function:

$$\sum_i \frac{(E_i - F_i)^2}{2\sigma_i^2} \quad \text{where } i \in (21, 32, 13)$$

with the constraint:

$$F_{21} + F_{32} + F_{13} = 0$$

Using Lagrange multipliers, the set of values which maximizes the likelihood is:

$$\hat{F}_{21} = E_{21} - \frac{\sigma_{21}^2}{\sigma_{21}^2 + \sigma_{32}^2 + \sigma_{13}^2}(E_{21} + E_{32} + E_{13}) = \frac{\sigma_{32}^2 + \sigma_{13}^2}{\sigma_{21}^2 + \sigma_{32}^2 + \sigma_{13}^2}E_{21} - \frac{\sigma_{21}^2}{\sigma_{21}^2 + \sigma_{32}^2 + \sigma_{13}^2}(E_{32} + E_{13})$$

$$\hat{F}_{32} = E_{32} - \frac{\sigma_{32}^2}{\sigma_{21}^2 + \sigma_{32}^2 + \sigma_{13}^2}(E_{21} + E_{32} + E_{13}) = \frac{\sigma_{21}^2 + \sigma_{13}^2}{\sigma_{21}^2 + \sigma_{32}^2 + \sigma_{13}^2}E_{32} - \frac{\sigma_{32}^2}{\sigma_{21}^2 + \sigma_{32}^2 + \sigma_{13}^2}(E_{21} + E_{13})$$

$$\hat{F}_{13} = E_{13} - \frac{\sigma_{13}^2}{\sigma_{21}^2 + \sigma_{32}^2 + \sigma_{13}^2}(E_{21} + E_{32} + E_{13}) = \frac{\sigma_{32}^2 + \sigma_{21}^2}{\sigma_{21}^2 + \sigma_{32}^2 + \sigma_{13}^2}E_{13} - \frac{\sigma_{13}^2}{\sigma_{21}^2 + \sigma_{32}^2 + \sigma_{13}^2}(E_{32} + E_{21})$$

The above estimators have no systematic bias, and there will be substantially no discrepancy between the free energy predictions from different paths. The method interprets the above estimators as the weighted average from the two paths. For example, the free energy difference between ligand $L_1$ and $L_2$, $F_{21}$, can be estimated from $E_{21}$, or from $-(E_{32} + E_{13})$, and the best estimator is a weighted average of the two predictions. The smaller the standard deviation for the computed free energy difference along the path is, the large the weight to the best estimator, and vice versa.

In addition, according to the above model, the hysteresis of the cycle closure, $E_{21} + E_{32} + E_{13} = \Delta$, is also Gaussian distributed with average of 0 and standard deviation of $s = \sqrt{\sigma_{21}^2 + \sigma_{32}^2 + \sigma_{13}^2}$. If the sum of the three calculated free energies deviates by more than 2s from 0, it is almost certainly ($P = 0.95$) that the calculations are not converged and the results are not reliable; if the sum of the three calculated free energies deviates by more than s from 0, it is highly probable ($P = 0.68$) that the calculation is not converged and the results may not be reliable. Thus the method determines whether the predictions are reliable.

In practical relative free energy calculations, due to the high computational expense, a given calculation is typically only performed for a single time, and the standard deviation cannot be estimated for each prediction. In this case, the method assumes that the standard deviation for each calculation is the same, for example 0.8kcal/mol for each path. Then if the discrepancy of the free energy results from two paths are larger than 1.4 kcal/mol, it indicates the calculation is not converged and probably not reliable. Under this assumption, the best free energy estimator has the following expression:

$$\hat{F}_{21} = E_{21} - \frac{1}{3}(E_{21} + E_{32} + E_{13}) = \frac{2}{3}E_{21} - \frac{1}{3}(E_{32} + E_{13})$$

$$\hat{F}_{32} = E_{32} - \frac{1}{3}(E_{21} + E_{32} + E_{13}) = \frac{2}{3}E_{32} - \frac{1}{3}(E_{21} + E_{13})$$

$$\hat{F}_{13} = E_{13} - \frac{1}{3}(E_{21} + E_{32} + E_{13}) = \frac{2}{3}E_{13} - \frac{1}{3}(E_{32} + E_{21})$$

In addition, under the assumption that the standard deviation for each calculation is the same, the hysteresis of the cycle closure itself provides an estimate of the error associated with each free energy prediction. As discussed above, the hysteresis $\Delta$ of the cycle closure is Gaussian distributed with average of 0 and standard deviation of $s = \sqrt{3}\sigma$. So the probability that a given calculation generates a hysteresis with value $\Delta$ for the cycle closure is:

$$P = \frac{1}{\sqrt{2\pi}\sqrt{3\sigma^2}}\exp(-\frac{-\Delta^2}{6\sigma^2})$$

The value of sigma which maximizes the above probability gives the maximum likelihood estimate of the standard deviation associated with each free energy calculation:

$$\hat{\sigma} = \frac{\Delta}{\sqrt{3}}$$

Note that in the above derivation, the free energies and the associated errors that the method estimates are the optimal estimates based on all the information gained from relative free energy calculations. If the underlying force field in the calculations has systematic bias, the perfectly converged relative free energy calculation results may still deviate from the experimental measured values, which is something that cannot be corrected based on the above analysis.
The method we describe above applies to more complicated cases with more members in the cycle closure and several cycles sharing the same edge. Here we illustrate the method for obtaining the best free energy estimators for the paths used in the FEP/MD and FEP/REST calculations for the 6 CDK2 ligands with crystal structures (Figure 2). The more general algorithm for any kinds of relative binding free energy calculation path is given in the following section.

$$\hat{F}_{21} = \frac{1}{2}E_{21} - \frac{1}{4}(E_{42} + E_{14}) - \frac{1}{4}(E_{32} + E_{13})$$

$$\hat{F}_{32} = \frac{5}{8}E_{32} - \frac{3}{8}(E_{13} + \frac{2}{3}E_{21} - \frac{1}{3}(E_{14} + E_{42}))$$

$$\hat{F}_{42} = \frac{5}{8}E_{42} - \frac{3}{8}(E_{14} + \frac{2}{3}E_{21} - \frac{1}{3}(E_{13} + E_{32}))$$

$$\hat{F}_{13} = \frac{5}{8}E_{13} - \frac{3}{8}(E_{32} + \frac{2}{3}E_{21} - \frac{1}{3}(E_{14} + E_{42}))$$

$$\hat{F}_{14} = \frac{5}{8}E_{14} - \frac{3}{8}(E_{42} + \frac{2}{3}E_{21} - \frac{1}{3}(E_{13} + E_{32}))$$

$$\hat{F}_{43} = \frac{1}{2}(E_{13} - E_{14}) + \frac{1}{2}(E_{42} - E_{32})$$

**Best free energy estimators using Bayesian statistics**

For a FEP calculation mutating from ligand $L_i$ to ligand $L_j$ with the theoretical free energy difference $F_{ji}$, suppose the calculated free energy $E_{ji}$ is Gaussian distributed with average $F_{ji}$ and standard deviate $\sigma_{ji}$ (the same model used for the Maximum Likelihood method). Then the probability density that the calculated free energy from one FEP calculation gives a value of $E_{ji}$ is,

$$\rho(E_{ji} \mid F_{ji}) = \frac{1}{\sqrt{2\pi}\sigma} \exp(-\frac{(E_{ji} - F_{ji})^2}{2\sigma^2})$$

Many different values of $F_{ji}$ can lead to the same calculated free energy $E_{ji}$ with different possibility. According to Bayesian statistics, before running the FEP calculation, the method has an estimate of the distribution (the prior distribution) of the free energy difference between the two ligands, $P_{prior}(F_{ji})$, and after it runs FEP calculation it adjusts the distribution based on the calculated result from FEP (the posterior distribution $P_{post}(F_{ji} \mid E_{ji})$.

Before running the FEP calculation, if no information is known about the relative binding free energy calculation, a reasonable assumption about the prior distribution of $F_{ji}$ would be that $F_{ji}$ is uniformly distributed between $-\infty$ to $+\infty$, ie

$$P_{prior}(F_{ji}) = 1 \text{ for } F_{ji} \in [-\infty, +\infty]$$

Note the prior distribution is not normalized.

If the FEP calculation gives a value of $E_{ji}$, then according to Bayesian statistics, the posterior distribution of $F_{ji}$ would be:

$$P_{post}(F_{ji} \mid E_{ji}) = \frac{\frac{1}{\sqrt{2\pi}\sigma}\exp\frac{(E_{ji} - F_{ji})^2}{2\sigma^2}}{\int_{-\infty}^{+\infty}\frac{1}{\sqrt{2\pi}\sigma}\exp\frac{(E_{ji} - F_{ji})^2}{2\sigma^2}dF_{ji}} = \frac{1}{\sqrt{2\pi}\sigma}\exp\frac{(E_{ji} - F_{ji})^2}{2\sigma^2}$$

For the case described above, using three ligands $L_1$, $L_2$, and $L_3$, the method performs FEP calculations forming a closed cycle, with the calculated free energy difference $E_{21}$, $E_{32}$ and $E_{13}$. Then according the Bayesian statistics, the posterior distribution of $(F_{21}, F_{32}, F_{13})$ is :

$$P_{post}(F_{21}, F_{32}, F_{13} \mid E_{21}, E_{32}, E_{13}) = \frac{(\frac{1}{\sqrt{2\pi}\sigma})^{2/3}\exp(-\frac{(E_{21} - F_{21})^2}{2\sigma^2} - \frac{(E_{32} - F_{32})^2}{2\sigma^2} - \frac{(E_{13} - F_{13})^2}{2\sigma^2})}{\int_{-\infty}^{+\infty}dF_{21}\int_{-\infty}^{+\infty}dF_{32}\int_{-\infty}^{+\infty}dF_{13}(\frac{1}{\sqrt{2\pi}\sigma})^{2/3}\exp(-\frac{(E_{21} - F_{21})^2}{2\sigma^2} - \frac{(E_{32} - F_{32})^2}{2\sigma^2} - \frac{(E_{13} - F_{13})^2}{2\sigma^2})}$$

$$P_{post}(F_{21}, F_{32}, F_{13} \mid E_{21}, E_{32}, E_{13}) \propto \exp(-\frac{(E_{21} - F_{21})^2}{2\sigma^2} - \frac{(E_{32} - F_{32})^2}{2\sigma^2} - \frac{(E_{13} - F_{13})^2}{2\sigma^2})$$

under the constrain that

$$F_{21} + F_{32} + F_{13} = 0$$

The pick of the posterior distribution is located at

$$\hat{F}_{21} = E_{21} - \frac{1}{3}(E_{21} + E_{32} + E_{13}) = \frac{2}{3}E_{21} - \frac{1}{3}(E_{32} + E_{13})$$

$$\hat{F}_{32} = E_{32} - \frac{1}{3}(E_{21} + E_{32} + E_{13}) = \frac{2}{3}E_{32} - \frac{1}{3}(E_{21} + E_{13})$$

$$\hat{F}_{13} = E_{13} - \frac{1}{3}(E_{21} + E_{32} + E_{13}) = \frac{2}{3}E_{13} - \frac{1}{3}(E_{32} + E_{21})$$

This confirms the results obtained from Maximum Likelihood method presented above.

**II. Programming a general implementation of the cycle closure-derived free energy estimate and error estimate invention**

For a set of L ligands, the method performs N relative free energy calculations which connect all the ligands with cycle closure(s). The particular embodiment of the invention described in this section yields the best predictions of the relative free energies among the ligands and the error bars associated with the predictions. The input is the N calculated free energy differences between the N pairs of ligand with relative free energy simulations performed, and the output of is the N predicted relative free energy differences and the associated error bars between the N pairs of ligands for which the relative free energy simulations were performed, and also the relative free energy differences between any pairs of ligands in the set and their associated error bars. The predicted relative free energies have no hysteresis, meaning the predicted relative free energy difference between any two ligands is independent of the paths connecting them, and thus are internally consistent.

In the example paths depicted in Figure 3, there are 11 relative free energy calculations for a set of 8 ligands which connects all the 8 ligands with several cycle closures. The input of the program is the 11 relative free energy calculations results for the 11 pairs of ligands, and the output is the predicted relative free energy differences for the 11 pairs of the ligands, their associated error bars, and also the relative free energy differences between any arbitrary pair of 8 ligands in the set and their associated error bars.

**Input:**

$\{E_i; L_j \rightarrow L_k\}$ where $i \in \{1,2,3,...N\}$

**Output:**

$\{F_i, e_i; L_j \rightarrow L_k\}$ where $i \in \{1,2,3,...N\}$
and $\{F_{ij}, e_{ij}\}$ where $i, j \in \{1,2...L\}$

**Algorithm**

Step 1: List the calculated free energies for all the relative binding free energy calculations performed, and their corresponding initial and final ligands.

What we get from this step is a list:
$\{E_i; L_j \rightarrow L_k\}$ where $i \in \{1,2,3,...N\}$
Here $E_i$ is the relative free energy simulation calculated free energy for the ith relative binding free energy calculation. The initial and final ligand for this relative binding free energy calculation is $L_j$ and $L_k$. This is the input of the program.

Step 2: List all the closed cycles in the relative binding free energy calculation paths and build up the Cycle Closure Connectivity Matrix (C3M) for the whole graph.

In this step, the method searches through all the ligand pairs and find all those pairs that form a closed cycle and convert this information into the connectivity matrix.
For example, in the ligand pair paths shown above, pairs 1, 2, and 5 form a closed cycle. Since free energy is a state function, the sum of the free energy along such a closed cycle should be 0. In other words

$$F_1 + F_2 - F_5 = 0$$

In the general case, the restrain that the free energy along a closed cycle can be written in the following general restrain function:

$$\sum_{i=1}^{N} M_i F_i = 0$$

where $F_i$ is the free energy difference between the two ligands on the two vertexes of the edge along the closed cycle, and $M_i$ is 1 if the edge is in the same direction as the closed cycle, -1 if the edge is in the opposite direction of the closed cycle, and 0 if the edge is not in the closed cycle.

In this step, the method lists all the closed cycles in the ligand pair paths, and all the restrains. In the example shown above, we have the following restrains for closed cycles:

$$F_1 + F_2 - F_5 = 0$$

$$F_3 + F_4 - F_5 = 0$$

$$F_4 - F_7 - F_6 = 0$$

$$F_1 + F_2 - F_4 - F_3 = 0$$

$$F_5 - F_7 - F_6 - F_3 = 0$$

$$F_1 + F_2 - F_7 - F_6 - F_3 = 0$$

$$F_9 + F_{11} - F_{10} = 0$$

In the general case, suppose there is a total number of M closed cycles in the ligand pair paths, then we have M restrain equations:

$$\sum_{i=1}^{N} M_{ji} F_i = 0 \text{ where } j \in \{1, 2...M\}$$

$M_{ji} = M_G$ is a $M \times N$ matrix and each row of the matrix represent a restrain equation for a closed cycle. We use $M_G$ to denote the Cycle Closure Connectivity Matrix (C3M) of the whole graph.
In the example shown above,

$$M_G = \begin{bmatrix} 1 & 1 & & & -1 & & & & \\ & & 1 & 1 & -1 & & & & \\ & & & 1 & & & -1 & -1 & \\ 1 & 1 & -1 & -1 & & & & & \\ & & -1 & & & 1 & -1 & -1 & \\ 1 & 1 & -1 & & & & -1 & -1 & \\ & & & & & & 1 & -1 & 1 \end{bmatrix}$$

$$\overbrace{\qquad\qquad\qquad}^{11\,columns}$$

Step 3: Decompose the whole graph into independent subgraphs.

In the relative free energy calculation graph, if there is no closed cycle connecting two subgraphs, then the predicted relative free energies among a subset of ligands are independent from the predicted relative free energies among the other subset of ligands. In the example shown above, the predicted relative free energies among the set of ligands [1,2,3,4,5] are independent from the relative free energies among the set of ligands [6,7,8] because there is no closed cycle connecting the two subgraphs. In this step, the whole graph is decomposed into each independent subgraph, and then each independent subgraph is solved sequentially.

Step 3a: Go over the each row of $M_G$, record the column number of the nonzero elements in each row, and put the column number into a list

$L_j = \{n_j; M_{jn_j} \neq 0\}$ where $j \in \{1,2...M\}$

In this step the method produces M lists. Each list contains the column number of the nonzero elements of each row of the C3M matrix. To make it clear for the following description, the set which contains the M lists generated in this step the raw list is called set $S_r$.

In the example shown above, this step of the method produces a raw list set which contains 7 lists

$L_1 = \{1,2,5\}$
$L_2 = \{3,4,5\}$
$L_3 = \{4,6,7\}$
$L_4 = \{1,2,3,4\}$
$L_5 = \{3,5,6,7\}$
$L_6 = \{1,2,3,6,7\}$
$L_7 = \{9,10,11\}$

Step 3b: Iterate through the second list to the last list in the raw list set $S_r$, and compare the elements contained in $L_j$ where $j \in \{2, 3,...m\}$ with the elements in the first list $L_1$. If they contain a common element (or a few elements), then merge all the elements in $L_j$ into the first list $L_1$, and delete $L_j$ from the raw list set; if they don't have any comment element, keep the list $L_j$ in the raw list set.

In the example shown above, $L_2$, $L_4$, $L_5$, $L_6$ contain common element(s) with $L_1$, so those lists are deleted in this step, and their elements are merged into $L_1$. What remains in the raw list set from this step is

$L_1 = \{1,2,3,4,5,6,7\}$
$L_3 = \{4,6,7\}$
$L_7 = \{9,10,11\}$

Step 3c: Repeat step 3b for those lists that have not been deleted in step 3b and merge the elements into $L_1$ if they contain common elements with $L_1$. Repeat this step until no more lists can be deleted.

In the example shown above, what remains in the raw list set $S_r$ from this step is

$L_1 = \{1,2,3,4,5,6,7\}$
$L_7 = \{9,10,11\}$

Step 3d: Move the first list $L_1$ in the raw list set $S_r$ into the final list set $S_f = \{L_1...\}$ and delete it from the raw list set.

In the example shown above, after this step in the final list set S

$S_f = \{L_1 = \{1,2,3,4,5,6,7\}\}$,
and there is one list left in the raw list set
$S_r = \{L_7 = \{9,10,11\}\}$

Step 3e: After step 3d, if there are more than one lists left in the raw list set, repeat step 3b to step 3d until there is only one list left.

In the example shown above, no action is need in this step, since there is only one list left in the raw list set after step 3d.

Step 3f: Move the last list in the raw list set $S_r$ into the final list set $S_f$. After this step, there are several lists in the final list set, and no list left in the raw list set. The lists in the final list sets do not contain any common elements.

In the example shown above, in the final list set we have
$S_f = \{L_1 = \{1,2,3,4,5,6,7\},L_2 = \{9,10,11\}\}$

Step 3g: Iterate through column number 1 to N. If the column number is in any list in the final list set, skip it, otherwise, add a list which contains only that column number into the final list set.

This is the final step in this process. After this step, the final list set contains several lists, and there is no common element between any of the lists. The column number 1 to N is in one and only one of the lists in the final list set.

In the example shown above, after this step:
$S_f = \{L_1 = \{1,2,3,4,5,6,7\},L_2 = \{9,10,11\},L_3 = \{8\}\}$

After this process, the whole graph is decomposed into each individual independent subgraph. Each independent subgraph contains the edges with the edges numbers listed in each list of the final list set $S_f$. These independent subgraphs do not have a shared edge, but they may have shared vertexes.

In the example shown above, the whole graph is decomposed into 3 independent subgraphs. The first subgraph contains 7 binding free energy calculation paths [E1, E2, E3, E4, E5, E6, E7], the second subgraph contains 3 relative binding free energy calculation paths [E9, E10, E11], and the third subgraph contains 1 relative binding free energy calculation path [E8]. These subgraphs are shown in Figure 4.

Step 4: Get the C3M for each independent subgraph $M_S$.

From Step 3, the whole connectivity graph is already decomposed into independent subgraphs, and the edges numbers of each subgraph is stored in the list in the final list set. In this step we get to get the C3M for each independent subgraph.

Take each list $L_i$ from the final list set $S_f$, suppose there are $N_i$ elements in the list. Search through all the rows of the C3M of the whole graph, $M_G$. For each row of $M_G$, search all those n elements whose column numbers are in the list $L_i$, and if any of them is not zero, put the n column elements of this row into $M_S^i$, the C3M for the subgraph corresponding to list $L_i$. Do this for all the rows of $M_G$ and for all the lists in the final list set $S_f$.

From this step, the C3M of the whole group, $M_G$, a $M \times N$ matrix, is decomposed into each independent C3M, $M_S^i$, a $M_i = N_i$ matrix, each corresponding to an independent each subgraph. The dimensionality of these matrixes satisfy the following properties:

$$\sum_i M_i = M$$

$$\sum_i N_i = N$$

After this step, the whole graph is decomposed into independent subgraphs, and the C3M of the whole graph, $M_G$, is also decomposed into C3M for each independent subgraph, $M_S^i$.

Note that, each row in $M_S^i$ represents a closed cycle in the path, and the connectivity matrix $M_S^i$ for some independent subgraph may contain zero elements, meaning there is no closed cycle in that subgraph.

In the above example, the C3Ms for the three independent subgraphs are:

$$M_S^1 = \begin{bmatrix} 1 & 1 & & & -1 & & \\ & 1 & 1 & -1 & & & \\ & & 1 & & & -1 & -1 \\ 1 & 1 & -1 & -1 & & & \\ 1 & 1 & -1 & & & -1 & -1 \end{bmatrix}$$

$$M_S^2 = \begin{bmatrix} 1 & -1 & 1 \end{bmatrix}$$

$$M_S^3 = \varnothing$$

Note that the $M_S$ for the third subgraph is empty.

Step 5: Reduce the C3M for each subgraph $M_S$ into independent matrix $M_{SR}$

In the C3M generated from step 4 for each subgraph, some rows are not independent from each other, meaning that the closed cycles listed in $M_S$ are not independent. For example, in the example shown above, for subgraph 1, there are three independent cycles, but all the six closed cycles are listed in the connectivity matrix $M_S^1$. Three of them are not necessary.

In this step, the C3M of each subgraph, $M_S^i$, is reduced into Matrix which only contains independent row vectors. We call the reduced matrix which only contains independent row vectors, RC3M (the Reduced Cycle Closure Connectivity Matrix), and denote it as $M_{SR}^i$.

There are many ways to reduce the matrix $M_S^i$ into $M_{SR}^i$. Here we list a simple one.

Step 5a: Put the first two rows of $M_S^i$ to form a new $2 \times N_i$ matrix M.

Step 5b: Calculate det $|MM^T|$. If the determent is not zero, then the two rows are independent; if the determent is zero, then the two rows are dependent, and delete the second row.

Step 5c: put the third row of $M_S^i$ into M, and repeat step 5b.

Step 5d: repeat step 5c for all the remaining rows in $M_S^i$.

After this step, $M_{SR}^i$ is obtained for each subgraph. The $M_{SR}^i$ only contains independent row vectors, meaning only independent closed cycles are listed in the matrix.

In the above example, the $M_{SR}$ for the first subgraph is:

$$M_{SR}^1 = \begin{bmatrix} 1 & 1 & & & -1 & & \\ & 1 & 1 & -1 & & & \\ & & 1 & & & -1 & -1 \end{bmatrix}$$

Note that, depending on the order of rows in C3M and the algorithm to reduce the matrix, the $M_{SR}$ for each subgraph is not unique, but the number of rows in $M_{SR}$ is independent on the order of rows and on the algorithm to do the reduction.

After this step, the reduced cycle closure connectivity matrix $M_{SR}$ is obtained for each independent subgraph. The $M_{SR}$ only contain independent cycles in each subgraph. In the following we will solve the cycle closure equation for each independent subgraph.

Step 6: Solve the cycle closure equation for each independent subgraph.

This step produces, for each subgraph, a set of optimal estimates of free energies for the $N_i$ pairs of ligands with no hysteresis, and it also maximizes the probability.

There are two different cases.

Case a: There is no closed cycle in the subgraph. Then the solution to this case is:

$$F_i = E_i$$
$$e_i = 0$$

In the example shown above, the third subgraph doesn't have any closed cycle. So the solution for the third subgraph is,

$$F_8 = E_8$$
$$e_8 = 0$$

Case b: There are m independent closed cycles in the subgraph.

In this case, for the subgraph corresponding to list $L_s$ in the final list set $S_f$, with n elements and the corresponding RC3M, $M = M_{SR}(m \times n$ matrix), the following function is minimized:

$$\sum_{i}^{n} (F_i - E_i)^2 \text{ where } i \in L_s$$

under the constrains that:

$$\sum_{i} M_{ji} F_i = 0 \text{ where } j \in \{1, 2...m\}, i \in L_s$$

Using the Lagrange multiplier, we may need to minimize the following functional:

$$L = \sum_{i}^{n} (F_i - E_i)^2 - \sum_{j}^{m} 2C_j (\sum_{i} M_{ji} F_i)$$

under the constrains that

$$\sum_{i} M_{ji} F_i = 0 \text{ where } j \in \{1, 2...m\}, \text{ and } i \in L_s$$

where $C_j$ are the coefficients of the Lagrange multipliers.

Solving the equation

$$\frac{\partial L}{\partial F_i} = 2(F_i - E_i) - \sum_{j}^{m} 2C_j M_{ji} = 0$$

the method establishes:

$$F_i = E_i + \sum_j^m C_j M_{ji}$$

$$= E_i + \sum_j^m M_{ji} C_j$$

$$= E_i + \sum_j^m M_{ij}^T C_j$$

Here $M_{ji}^T$ is the transpose of $M_{ji}$.

Written in the Matrix formulation, where

$$\vec{E} = \begin{Bmatrix} E_1 \\ E_2 \\ \cdot \\ \cdot \\ \cdot \\ E_n \end{Bmatrix}, \vec{F} = \begin{Bmatrix} F_1 \\ F_2 \\ \cdot \\ \cdot \\ \cdot \\ F_n \end{Bmatrix}, \vec{C} = \begin{Bmatrix} C_1 \\ C_2 \\ \cdot \\ \cdot \\ \cdot \\ C_m \end{Bmatrix}$$

and

$$M = \begin{bmatrix} M_{11} & M_{12} & \cdot & \cdot & \cdot & M_{1n} \\ M_{21} & & & & & \\ \cdot & & & & & \\ \cdot & & & & & \\ \cdot & & & & & \\ M_{m1} & M_{m2} & \cdot & \cdot & \cdot & M_{mn} \end{bmatrix}$$

yielding the following two sets of equations:

$$(1): \vec{F} = \vec{E} + M^T \vec{C}$$

$$(2): M\vec{F} = \vec{0}$$

Plug equation (1) into equation (2) we get the following equation:

$$(3): MM^T \vec{C} = -M\vec{E}$$

Both the RC3M matrix M and the vector E are known, so the equation can be solved to get the coefficients vector C. After the coefficients are solved, equation (1) yields the best free energy estimators for the n pairs of simulations.

Step 7: Get the error estimates for each possible relative binding free energy calculation path

According to the hypothesis introduced above, the sum of the relative free energy calculations along a closed cycle is Gaussian distributed with standard deviation $\sqrt{n}s$ where n is the number of members on the closed cycle. The error for each calculated difference between a pair of ligands on this closed cycle is $\dfrac{\Delta}{\sqrt{n}}$, where $\Delta$ is the hysteresis of the closed cycle.

For each pair of ligands on the closed cycle, the error for the computed free energy difference between the pair of ligands on the closed cycle can be estimated by the hysteresis of that cycle.

Written in a more reduced form, for each row of the C3M, $M_S$ (Note here is the unreduced cycle closure connectivity matrix), the following error estimate can be assigned to each member on the cycle:

$$(4): e_{ji} = |M_{ji}| \frac{\sum_i^n E_i}{\sqrt{\sum_i^n |M_{ji}|}}$$

After doing the error calculation according to equation (4) for all closed cycles, that is for all the rows in $M_S$, the final error estimate for relative binding free energy estimate *i* is chosen as the maximal of the estimates from all rows. In other words:

$$(5): e_i = Max\{e_{ji}; j \in \{1,2...m\}\} = Max\{|M_{ji}| \frac{\sum_i^n E_i}{\sqrt{\sum_i^n |M_{ji}|}}; j \in \{1,2...m\}\}$$

After Step 6 and Step 7, we get the best free energy estimates for the n relative free energies and also their associated error bars for each independent subgraph.

Step 8: Report the estimate of the free energy difference between any pairs of ligands in the set and their associated error bars

In the previous steps, the method has already provided best free energy estimates and their associated error bars for the n pairs of ligands in each independent subgraph. Merging these date together from all independent subgraphs, produces Output:

$$\overrightarrow{F_i} = \begin{Bmatrix} F_1 \\ F_2 \\ \cdot \\ \cdot \\ \cdot \\ F_N \end{Bmatrix} \text{ and } \vec{e} = \begin{Bmatrix} e_1 \\ e_2 \\ \cdot \\ \cdot \\ \cdot \\ e_N \end{Bmatrix}$$

For any pairs of ligands that are not listed in the above, the method lists all the possible paths on the graph that can connect the two ligands, and then sum over the free energies on each edge of the paths and also the errors. The free energy is independent of the path, but the errors may dependent on the path. The maximal of all the errors to report can be written in a concrete formula:

$$F_{ij} = \sum_k I_k F_k$$

$$e_{ij} = Max \{ e_{ij}^k = \sqrt{\sum_k e_k^2} \}$$

where

$$I_k = \begin{cases} 1 \; ; & \text{when } F_k \text{ is in the same direction as the path} \\ -1 \; ; & \text{when } F_k \text{ is in the opposite direction as the path} \end{cases}$$

and the maximal is taken for all possible paths which connects ligand $L_i$ and ligand $L_j$ Note that, in this step, the free energy estimates and errors are only calculated for those pairs of ligands where free energy calculations are not performed (not directly connected in the graph). The free energy estimates and errors for those pairs of ligands where free energy calculations are performed are calculated in previous steps.

To one skilled in the art, it should be apparent in the above that many different approaches may be taken to develop a computer implementation of a probabilistic model of the probable free energy differences and their errors that give rise to the hysteresis(es) observed for closed cycle(s); and to make optimal predictions of the free energy differences and their errors given such a probabilistic model. For example without limitation, matrix or graph methods may be used to create the required data structure, matrix algebra or graph theory methods may be used to construct the probabilistic model, and maximum likelihood or Bayesian methods may be used to parameterize the model to be consistent with the hysteresis(es). The matrix algebra formulation in the preceding section is only a particular embodiment of the invention, and can be readily generalized in a number of ways, including assuming the most probable error of the legs of the pathway is additive with the Bennett error, the Bootstrap error, or both. Likewise it might be generalized to use other distributions aside from the Gaussian distribution, such as without limitation, the Lorentz, Levy, or Rayleigh, delta and uniform distributions.

As such, the variety of approaches that might be considered to construct a particular probabilistic model of the probable free energy differences and their errors that give rise to the hysteresis(es) observed for closed cycle(s); and to make optimal predictions of the free energy differences and their errors given such a probabilistic model would be interesting embodiments of the invention described herein.

**Illustrative examples of applications of the invention to various particular relative binding free energy calculations to various particular receptors and ligands**

*FEP/REST relative free energy calculation algorithm*

The invention improves the accuracy and reliability of relative free energy calculations (for example explicit solvent model calculations). Such accuracy is particularly difficult to achieve if the protein residues surrounding the binding pocket move significantly when different ligands bind, or the two ligands adopt different binding modes or manifest one or more degenerate binding modes.[4,11,19] In such cases, sampling all relevant conformations improves the accuracy of estimates of the free energy difference, yet the transition times to interchange between the different conformations may be too large to be accessible on the time scale of most FEP/MD simulation lengths.

Here, we present a particular set of relative binding free energy calculations that illustrates the utility of the method using a recently developed relative free energy calculation method, FEP/REST. FEP/REST, introduced by Wang et al,[12] incorporates the enhanced sampling method, REST, into FEP calculations through an efficient $\lambda$-hopping protocol, to sample relevant local structural rearrangements in relative protein-ligand binding affinity calculations within easily accessible simulation times. We also present more routine FEP/MD relative free energy calculations, to similarly illustrate the utility of the invention, independent of the particular chosen relative binding free energy calculation methodology.

FEP involves calculating the free energy difference between two systems by alchemically transforming between the systems in a series of discrete steps represented by $\lambda$ values, where $\lambda$ varies from 0 for the initial state to 1 for the final state. FEP/REST modifies the potential energy for a localized region surrounding the binding pocket (which we call the 'hot' region) which may include protein residues as well as the ligand. For the intermediate $\lambda$ windows, the potential energy for the localized region is scaled by a factor less than 1. In this way, energy barriers are lowered, enabling the efficient sampling of the different conformations in these intermediate $\lambda$ windows, and the different conformations are propagated to the end states through the Hamiltonian replica exchange method.[15,22,23] We call the region where the potential energy is scaled the 'hot' region, since the local contributions to the potential energy have been scaled to smaller values such that the effective temperature of that region is higher, although this analogy does not carry over to the particles actually having higher kinetic energies in the simulation. The details about the FEP/REST algorithm are described in the FEP/REST work by Wang et al.[12].

The 'hot' region for REST enhanced sampling is configured here to include the ligand functioned group mutated in the SAR studies. If the ligand functional group is attached to an aromatic ring, the aromatic ring is also included in the 'hot' region, allowing the ring to flip through REST enhanced sampling. The effective temperature profile for REST 'hot' region is calculated according to equation 9 of reference 26[24], setting the expected acceptance ratio of replica exchange to be 0.3, an optimal value reflecting the trade-offs between the number of replicas needed and exchange efficiency. A total of 12 $\lambda$ windows are used for the both normal FEP and FEP/REST calculations. The electrostatic interactions that are unique to the initial state were turned off before the Lennard-Jones (LJ) interactions, and the LJ interactions that are unique for the final state were turned on before the electrostatic interactions. The bonded interactions are smoothly turned from the initial state to the final state. The core of the LJ interactions was softened to avoid the singularities and instabilities in the simulations.[25] In our current simulations, the benzene ring on which substituents were varied in the SAR studies,[16] are included in the 'hot' region, and the highest effective temperature with a total of 12 $\lambda$ windows achieved is roughly 900K.

### Details of the various FEP/REST and FEP/MD relative free energy calculations:

The ligand functional group mutation module implemented in Desmond[26,27] was used to set up the calculations.[28] The OPLS2005 force field[29,30] was used for the proteins and the ligands along with the SPC[31] water model. The starting structures for the simulations were taken from the PDB structures with IDs 1h1q, 1h1r, 1h1s, 1oiu, 1oi9, and 1oiy.[16] Whenever multiple conformations were present in the PDB, we selected the first conformation for use in the simulations. The protein was prepared using the Protein Preparation Wizard[32,33] during which the protonation states were assigned assuming a pH of 7.0.[34] Cyclin A was retained with CDK2 and treated as part of the receptor for all calculations. $Na^+$ or $Cl^-$ ions were added to maintain electric neutrality. The systems were relaxed and equilibrated using the default Desmond relaxation protocol, which involves a series of minimizations and short molecular dynamics simulations. A total of 12 $\lambda$ windows were used for the both the normal FEP and FEP/REST calculations. The production stage lasted 2 ns for the complex simulations and 5 ns for the solvent simulations. Replica exchanges between neighboring lambda windows were attempted every 1.2ps. The production stages were sampled using both FEP/MD and FEP/REST. The Bennett acceptance ratio method (BAR)[35] was used to calculate the free energy. Errors were estimated for each free energy calculation using both bootstrapping[19,36] and the BAR analytical error prediction,[19,36,37] and the larger of the two errors was reported.

### Assessing the consistency and reliability of the predictions by way of a particular application of the invention

One can rank order the relative binding affinities for the whole set of ligands in relative protein-ligand binding affinity FEP calculations, most simply, by performing all FEP simulations from a common reference ligand. In addition, if one obtains a fully converged result for each calculation, the final predictions are, theoretically, independent of the paths chosen to do the relative binding free energy calculations. For example, for three ligands A, B, and C, there are two strategies to obtain the relative binding affinity between ligand A and ligand C: (1) sample the path directly from A to C; or (2) sample the path in two steps, from A to B and B to C, and then sum the two obtained free energies. Theoretically, the final converged free energy estimate should be the same from the above two methods. In practice, however, due to the errors in each calculation-including unbiased errors due to the random fluctuations in the sampling of the phase space which may be characterized by the Bennett error bound, and biased error due to a *systematically* incomplete sampling of the phase space which would not be characterized by the Bennett error bound-both the variances and the average values from the above two paths are usually somewhat different.[38] Given these limitations, it is important to assess the reliability of the results obtained.

To gain additional information about the consistency and the reliability of the predictions, we include cycle closures for all the paths. In the example given above, if the results from the two paths agree to within a specified threshold, it indicates that the predictions are consistent, and likely reliable within the limits of the employed force field. However, if the results from the two paths differ by more than some threshold value, it may indicate that the results may not be converged and the predictions may not be reliable. The threshold value depends on the number of members in each cycle and the error we expect for leg in the cycle. In this particular embodiment of the invention rigorously assess the reliability of the predictions and how to adaptively assign an appropriate threshold value for each cycle. In addition, once we include cycle closures in the path, there are multiple independent ways to calculate the free energy difference between two states. In this particular embodiment of the invention, optimal predictions from the multiple free energy estimates obtained along the closed cycles are also reported. The invention in this embodiment also directly provides information about the convergence error associated with the free energy calculations from the hysteresis of each cycle.

The methods discussed herein are performed by a computer system programmed to perform those steps. In Figure 15, the system includes computer(s) 110, data stores 120 and program modules 130. The computer instructions are stored in one or more non-transient computer-readable media. Figure 16 shows steps in the method including a listing of calculated free energies for all the free energy calculation paths (302), listing all closed cycles and building cycle closure connectivity matrix (304) and decomposition the entire graph into independent subgraphs (306).

Below we discuss certain specific examples and present related tables as follows:

Table 1: The FEP/MD and FEP/REST predicted relative binding free energies for the five ligands with crystal structures compared to the reference ligand 1h1q.

Table 2: The final free energy predictions for the 6 ligands with crystal structures from FEP/MD and FEP/REST calculations using the formula derived by the embodiments of the invention.

Table 3: The structures of CDK2 inhibitors and their compound # studied using FEP/REST.

Table 4: The FEP/REST predicted relative binding free energies for the additional 10 compounds without crystal structures.

Table 5: The final free energy predictions for the whole set of ligands using the formula derived by embodiments of the invention.

Table 6: FEP/REST results for the 8 relative binding free energy calculation paths of the jnk1 ligands.

Table 7: The predicted binding affinities and their associated errors for the 7 ligands binding to jnk1.

Table 8: FEP/REST results for the 7 relative binding free energy calculation paths for the BACE ligands.

Table 9: The predicted binding affinities and their associated errors for the 6 ligands binding to BACE.

Table 10: MC/FEP results for the 9 relative binding free energy calculation paths of scytalone dehydratase ligands.

Table 11: The predicted binding affinities and their associated errors for the 3 ligands binding to scytalone dehydratase.

Table 12: The cycle closure predicted free energy differences among the four states depicted on figure 14, and their associated errors.

## Results and Discussions

### *Performance on ligands with crystal structures*

FEP/MD and FEP/REST simulations were carried out to predict the relative binding affinities for the six ligands displayed in Figure 5 binding to the CDK2-cyclin A receptor. These are the only six ligands with crystal structures available for each holo complex, with PDB IDs 1h1q, 1h1r, 1h1s, 1oi9, 1oiu, and 1oiy,[16] and experimental binding affinity data for these ligands came from the same publication using the same method. Thus, the calculations reported here are essentially purely a scoring exercise, since the binding modes of ligands are known from the crystallography. In these crystal structures, the CDK2 receptor conformation is essentially the same regardless of which ligand is bound. Hence, we did not expect the sampling of protein conformational changes to significantly limit simulation convergence. In the calculations, we chose to use the protein conformation and ligand from 1h1q as the starting structure for all five perturbations (drawn with blue arrows in Figure 5), since all other ligands in the set are generated by adding different substituents on the benzene ring of ligand 1h1q.

### *Comparison between FEP/MD and FEP/REST results*

The FEP/MD and the FEP/REST results for the relative binding free energy calculation paths among the ligand with crystal structures are given in Table 1. The correlation of the predictions with experimental binding affinity data is displayed in Figure 6. Despite the ~3.5 kcal/mol range of experimental binding affinities, FEP/MD predicts that the five compounds (excluding the reference compound 1h1q) have almost the same binding affinities, resulting in an $R^2$ value of 0.32. By comparison, the FEP/REST results are highly correlated with the experimental values ($R^2$=0.91), and the deviations of the FEP/REST results from the experimental values are less than 0.8 kcal/mol for all of the ligand pairs.

From comparisons of the FEP/MD and FEP/REST results, it appears that the predictions for compounds 1oi9, 1oiu, and 1ioy from the two methods are essentially the same, and that the main reason for the improved correlation with the experimental results comes from the better binding affinity estimates for ligands 1h1s and 1h1r using FEP/REST. Below we will discuss why FEP/REST significantly outperforms FEP/MD for these two cases.

A review of the FEP/MD and FEP/REST trajectories for ligand 1h1r showed that the 2-chloro substituted benzene ring flips several times in the FEP/REST trajectory, while that ring remains trapped in the initial conformation in the FEP/MD trajectory. The initial conformation, corresponding to one of the conformations in the crystal structure, and the alternative flipped conformation, observed in the FEP/REST calculation, are displayed in Figure 7. Interestingly, for ligand 1h1r, the electron density in the deposited X-ray crystal structure also indicated the presence of an alternative ligand binding mode (Figure 7 right panel) that corresponds to the alternate one found in the FEP/REST calculations.

For 1h1s, in addition to the binding mode modeled in the crystal structure, another conformation is sampled in the FEP/REST trajectory, but not in the FEP/MD simulations. Figure 8 displays the binding mode found in the crystal structure and the alternative binding mode sampled in the FEP/REST calculation. In the crystal structure, the sulfonamide group makes two hydrogen bonds (HB) with Asp86: one with the backbone and another with the side chain. In the alternative binding mode sampled in the FEP/REST simulation, the sulfonamide group rotates about 120° around the CS bond relative to the crystal structure. The protein residues surrounding the binding pocket, particularly Gln85 and Asp86, rearrange accordingly and two hydrogen bonds are formed between the sulfonamide group and the protein residues: one with the Asp86 side chain and the other with the Gln85 side chain. The B factors of these few residues surrounding the binding pocket and the ligand in the X-ray crystallography indicate these groups are quite mobile.[16] Therefore, it is quite possible that the ligand can switch between the two binding modes in solution. In the FEP/REST simulation trajectory, the occupancy ratio between the binding mode observed crystallographically and the alternative binding mode is roughly 60:40, in favor of the crystallographically determined mode. However, sampling this alternative binding mode appears to be very important for the correct prediction of the binding affinity, and is responsible for the greater efficacy of FEP/REST in accurately computing the relative binding free energy of this species compared with FEP/MD. Interestingly, the invention allows this to be determined without reference to the experimental data, since the computed errors associated with the FEP/MD computed by this particular embodiment of the invention to be high (1.01 kcal/mol for 1oiu and 1h1s, and ~0.7 kcal/mol for 1oiy, 1oi9 and 1h1s), where as the computed errors associated with the FEP/REST are computed by the invention to be low (between 0.2~0.4 kcal/mol for all the ligands).

### *Assessing the consistency and reliability of the relative free energy calculations with a particular embodiment of the invention*

To assess the consistency and reliability of the FEP/REST predictions, we performed three more simulations forming three cycle closures in the relative binding free energy calculation paths (Figure 5). The FEP/REST results for the three paths forming these cycles are given in the last three rows of Table 1. The final predictions for the binding affinities of these ligands determined by way of a particular embodiment of the invention are given in Table 2. The cycles are closed self consistently with discrepancies less than 0.8 kcal/mol from different paths for all three cycles, indicating that the free energy is well converged and the predictions are highly reliable within the limits of the underlying force field.

However, even though the cycles are well closed and the free energies calculated from different paths agree to within a rather tight threshold value, they are not exactly the same. Thus, for the same pair of ligands, we have multiple different estimates for the free energy differences depending upon how one traverses the paths between the ligand. One is then left with the question of how to best incorporate all the information that is generated in the calculations to obtain the statistically best estimates for the free energies? We use a particular embodiment of the invention to determine the optimal estimates of the free energies and the errors associated with these predictions. Here, all the cycles are well closed, and the final predictions with cycle closures agree well with the above predictions without cycle closures, further validating the high reliability of these calculations. The deviation between the final predictions and the experimental results are all less than 0.7 kcal/mol, and they have very high correlation with the experimental data ($R^2$=0.92).

In contrast when this particular embodiment of the invention was applied to similar closed cycles computed with the FEP/MD relative free energy calculations, the hysteresis of the closed cycles involving ligand 1h1r and 1h1s are very large (as large as 1.75 kcal/mol and 1.35 kcal/mol respectively), indicating the FEP/MD calculations are not converged. Using the cycle closure free energy estimate by way of this particular embodiment of the invention, the errors for these FEP/MD free energy calculations are much large (1.01 kal/mol and 0.68 kal/mol respectively) than the FEP/REST results (errors of 0.35 kcal/mol and 0.25 kcal/mol respectively).

### *Performance on ligands without crystal structure*

To further validate our model, additional FEP/REST calculations were performed for another 10 ligands binding to the same receptor without crystal structure available for the holo complex. These 10 ligands were randomly selected from the same publication by Handcastle.[16] The binding affinities were measured using the same method as the above-mentioned ligands with crystal structures and they conserve the same core. The structures of the ligands and their corresponding compound numbers as in the original publication[16] are given in Table 3. The FEP/REST predicted relative binding affinities for these ligands are reported in Table 4 along with the experimental data. Cycle closures are constructed in the paths for these ligands (Figure 9), and the hysteresis of the cycle closures are all less than 1.0 kcal/mol, indicating the calculations are highly converged. The final predicted relative binding affinities by way of this particular embodiment of the invention are reported in Table 5. For ligands with cycle closures constructed in the relative binding free energy calculation paths, the errors associated with the free energy predictions are also reported in Table 5. The correlation between the predicted relative binding affinities and the experimental data for the whole set of ligands (6 ligands with crystal structure and the 10 ligands without crystal structure) is displayed in Figure 10. It is clear this particular embodiment of the invention when applied to the FEP/REST free energy calculation data performs well for these ligands without crystal structures, further demonstrating the transferability of the approach. The average deviation between the predicted binding affinities compared to experimental data is 0.44 kcal/mol, and the $R^2$ value is 0.82.

## Conclusions

Above, we have illustrated that the method can be applied in multiple embodiments to the recently developed free energy calculation FEP/REST method as well as the more routinely used FEP/MD free energy calculation method to calculate the relative binding affinities between a series of congeneric ligands binding to the CDK2-cyclin A receptor. The invention facilitated comparisons between FEP/REST and FEP/MD free energy calculation predictions. While the more routine FEP/MD method performed poorly in rank-ordering the relative binding affinities for the six ligands with crystal structure, the invention provided value indicating the error associated with the FEP/MD predictions was high. In contrast, he FEP/REST calculated relative binding affinities were in excellent agreement with the experimental data, and shown to be converged by application of the invention. Application of the invention to relative free calculations performed by way of FEP/REST method on a larger data set that includes 10 ligands without crystal structures further verified the invention's ability to correctly rank order the relative binding affinities of congeneric ligands.

The invention provides a means of assessing the consistency and reliability of free energy calculations using information derived from cycle closure calculations associated with any type of relative free energy calculation procedure. This invention provides clear criteria by which to judge the consistency and reliability of the predictions, as well as optimal estimates of the free energies with well-formed error bounds. Application of this invention to the CDK2-cyclin A series indicates our predictions using FEP/REST relative free energy calculations are highly reliable, which is also demonstrated by the excellent agreement of the predictions with the experimental data. Likewise the invention properly determined the FEP/MD predictions are less reliable.

We include additional applications of various embodiments of the invention to other systems and other relative binding free energy calculation in the generality and utility of the invention.

## Additional applications of various embodiments of the invention to a variety of systems using a variety of relative binding free energy calculation schemes

### I. Application of an embodiment of the invention to JNK1 kinase

FEP/REST relative binding free energy simulations were performed for a subset of ligands binding to jnk1 receptor with two cycle closures in the relative binding free energy calculation paths (Fig 11). The ligand numbers for the subset of ligands are [2,6,7,9,10,12,13], and the 8 FEP/REST results for the 8 relative binding free energy calculation paths are listed in the Table 6.

Using cycle closure best free energy estimators and error estimators, the binding affinities for the 7 ligands and their associated error bars are reported in Table 7.

Note that, the cycle closure estimates can not correct the systematic errors in the force field of the simulations. So, the predicted binding affinities for two of the ligands (ligand 12 and 13) deviates by more than 1.5 kcal/mol compared to experimental values. The deviations may either due to the error in the force field, or in the uncertainties in the experimental measurements. The overall correlation between the predicted results and the experimental values is very good, though.

### II. Application of an embodiment of the invention to BACE

FEP/REST simulations are performed for a subset of ligands binding to BACE receptor with two independent cycle closures in the raltive binding free enery calculation paths (Shown in Figure 12). The ligand numbers for the subset of ligands are [4j, 4o, 4p, 17d, 17g, 17h], and the 7 FEP/REST results for the 7 relative binding free energy calculation paths are listed in the Table 8.

Using cycle closure best free energy estimators and error estimators, the binding affinities for the 6 ligands and their associated error bars are reported in table 9.

Note again, the binding affinity for ligand 17h is over predicted by about 2 kcal/mol, but the overall correlation between the predicted results and the experimental values is very good.

### III. Application of an embodiment of the invention to FEP/MC relative binding free energy calculations of ligands binding to scytalone dehydratase

The relative binding affinities between 3 ligands binding to scytalone dehydratase with and without water bound in the active site are calculated using FEP/MC.[39] The relative binding free energy calculation paths for these simulations with 4 independent cycle closures are displayed in Fig 13. The calculated binding affinities for the relative binding free energy calculations are reported in Table 10.

The hysteresis of the cycle [L1(H2O), L2(H2O), L3(H2O)] is 4.3 kcal/mol, much larger than 1.4 kcal/mol, indicating these calculations are not converged, and the calculated binding affinities inside this cycle also have large deviations from the experimental data. Similarly, for the cycle [L3(H2O), L3, L2, L2(H2O)], the hysteresis is 1.5 kcal/mol, larger than 1.4 kcal/mol, indicating these calculations are not reliable either.

On the other hand, the hysteresis of the other cycles, [L1(H2O), L1, L3, L3(H2O)] and [L1, L2, L3], are very small (0.2 kcal/mol and 0.5 kcal/mol respectively), indicating the calculated are reliable. The final predicted relative binding affinities among the three ligands with the cycle closure estimates described above is reported in Table 11.

### IV. Application of an embodiment of the invention to test particle insertion relative binding free energy sampling for model enclosures

The relative binding free energies between a united atom methane and model enclosures with and without charge are calculated using test particle insertion method.[40] The free energies of charging the model enclosure with and without the bound methane is also calculated using FEP/MD sampling. The relative binding free energy calculation paths form a closed cycle (Figure 14) and the associated free energies for each edge on the cycle is reported in there.

Using cycle closure analysis described above, the predicted free energy difference between the four states depicted on Fig 16 and their associated errors are reported in Table 12.

TABLES.

**Table 1:** The FEP/MD and FEP/REST predicted relative binding free energies for the five ligands with crystal structures compared to the reference ligand 1h1q. The results for the three relative binding free energy calculation paths forming 3 cycle closures for the 6 ligands with crystal structures are also include in the table. The errors for the calculated free energies using BAR analytical error estimation are also included in the table. The FEP/MD predicted results have large deviations from the experimental data for ligands 1h1r and 1h1s **(bold** in the table), while the FEP/REST predicted results all agree well with the experimental data. Free energies are reported in kcal/mol unit.

| Ligand | ΔΔG(EXP) | ΔΔG(FEP/MD) | Δ(|FEP/MD-EXP|) | ΔΔG(FEP/REST) | Δ(|FEP/REST-EXP|) |
|---|---|---|---|---|---|
| 1h1q→Mh1r | 0.51 | -1.29±0.09 | **1.80** | 0.14±0.11 | 0.37 |
| 1h1q→1h1s | -3.07 | -1.64±0.27 | **1.43** | -2.72±0.28 | 0.35 |
| 1h1q→1oi9 | -1.56 | -1.83±0.14 | 0.27 | -1.70±0.16 | 0.14 |
| 1h1q→1oiu | -0.91 | -1.68±0.25 | 0.77 | -1.71±0.26 | 0.80 |
| 1h1q→1oiy | -1.61 | -1.69±0.22 | 0.08 | -1.92±0.23 | 0.31 |
| 1h1r→1oiu | -1.41 | 1.36±0.22 | **2.77** | -1.25±0.25 | 0.16 |
| 1oi9→1h1s | -1.51 | 0.14±0.29 | **1.65** | -0.81±0.30 | 0.70 |
| 1oi9→1oiy | -0.04 | 1.44±0.24 | **1.48** | 0.49±0.24 | 0.53 |

**Table 2:** The final free energy predictions for the 6 ligands with crystal structures from FEP/MD and FEP/REST calculations using the formula derived by the embodiments of the invention. The error estimates using the formula derived by embodiments of the invention for the final free energy prediction using cycle closure are also included in the table. Ligand 1h1q is used as the reference, so the deviation for ligand 1h1q is 0 by design. Note, a cycle closure error bar is not reported for ligand 1h1q since it is the reference ligand used for this work, and its binding free energy is set identically equal to its experimental value for plotting purposes. Free energies are reported in kcal/mol unit.

| | ΔG(EXP) | ΔG(FEP/MD) | A(|FEP/MD-EXP|) | FEP/MD_ Err (cycle_ closure) | ΔG(FEP/REST) | Δ(|FEP/REST-EXP|) | FEP/REST _Err(cycle_ closure) |
|---|---|---|---|---|---|---|---|
| 1h1q | -8.18 | -8.18 | -- | | -8.18 | -- | -- |

(continued)

| | ∆G(EXP) | ∆G(FEP/ MD) | A(\|FEP/ MD-EXP\|) | FEP/MD_ Err (cycle_ closure) | ∆G(FEP/ REST) | ∆(\|FEP/R EST-EXP\|) | FEP/REST _Err(cycle_ closure) |
|---|---|---|---|---|---|---|---|
| 1h1r | -7.67 | -10.05 | 2.38 | 1.01 | -8.25 | 0.58 | 0.35 |
| 1h1s | -11.25 | -10.00 | 1.25 | 0.68 | -10.91 | 0.34 | 0.25 |
| 1oi9 | -9.74 | -10.32 | 0.58 | 0.75 | -10.11 | 0.37 | 0.41 |
| 1oiu | -9.08 | -9.85 | 0.77 | 1.01 | -9.71 | 0.63 | 0.35 |
| 1oiy | -9.79 | -9.38 | 0.41 | 0.75 | -9.86 | 0.07 | 0.41 |

**Table 3**: The structures of CDK2 inhibitors and their compound # studied using FEP/REST.

| | | | |
|---|---|---|---|
| Compound: 17 | Compound: 20 | Compound: 21 | Compound: 22 |
| Compound: 26 | Compound: 28 | Compound: 29 | Compound: 30 |
| Compound: 31 | Compound: 32 | Compound: 1h1q | Compound: 1h1r |
| Compound: 1h1s | Compound: 1oi9 | Compound: 1oiu | Compound: 1oiy |

**Table 4:** The FEP/REST predicted relative binding free energies for the additional 10 compounds without crystal structures. The errors for the calculated free energies using BAR analytical error estimation are also reported in the table. Free energies are reported in kcal/mol unit.

| Ligand | ∆∆G(EXP ) | ∆∆G(FEP/REST ) | ∆(\|FEP/REST-EXP\|) |
|---|---|---|---|
| 1h1q→17 | 1.14 | 0.19±0.11 | 0.95 |
| 1h1q→20 | -0.54 | -0.52±0.18 | 0.02 |
| 1h1q→21 | 0.35 | 0.64±0.15 | 0.29 |
| 1hq→22 | 0.42 | 0.44±0.15 | 0.02 |
| 1h1q→26 | -0.25 | -1.39±0.14 | 1.14 |
| 1h1q→31 | -1.36 | -0.57±0.19 | 0.79 |

(continued)

| Ligand | ΔΔG(EXP ) | ΔΔG(FEP/REST ) | Δ(|FEP/REST-EXP|) |
|---|---|---|---|
| 1h1s→28 | 0.14 | 0.14±0.20 | 0.00 |
| 1h1s→30 | 1.44 | 1.53±0.15 | 0.09 |
| 1h1s→32 | 1.50 | 0.78±0.17 | 0.72 |
| 28→29 | 1.23 | 1.05±0.18 | 0.18 |
| 1h1r→17 | 0.63 | 0.13±0.11 | 0.50 |
| 21→22 | 0.07 | 0.62±0.24 | 0.55 |
| 1h1s→29 | 1.37 | 2.19±0.19 | 0.82 |
| 30→29 | -0.07 | -0.27±0.19 | 0.20 |
| 1oiu→26 | 0.65 | 1.99±0.19 | 1.34 |

**Table 5:** The final free energy predictions for the whole set of ligands using the formula derived by embodiments of the invention. The error estimates using the embodiments of the invention for the final free energy prediction using cycle closure are also included in the table. Ligand 1h1q is used as the reference, so the deviation for ligand 1h1q is 0 by design. Note, a cycle closure error bar is not reported for ligand 1h1q since it is the reference ligand used for this work, and its binding free energy is set identically equal to its experimental value for plotting purposes. Cycle closure error bars are also not reported for ligands 20, 31 and 32, since those species were technically challenging to connect to other ligands other than the reference ligand, as required to form closed cycles for the error. Free energies are reported in kcal/mol unit.

|  | ΔG(EXP) | ΔG(FEP/REST) | Δ(FEP/REST -EXP|) | Err(cycle_ closure) |
|---|---|---|---|---|
| 17 | -7.04 | -8.06 | 1.02 | 0.26 |
| 20 | -8.72 | -8.70 | 0.02 | -- |
| 21 | -7.83 | -7.81 | 0.02 | 0.47 |
| 22 | -7.76 | -7.47 | 0.29 | 0.47 |
| 26 | -8.43 | -8.65 | 0.22 | 0.69 |
| 28 | -11.11 | -10.51 | 0.60 | 0.58 |
| 29 | -9.88 | -9.20 | 0.68 | 0.58 |
| 30 | -9.81 | -9.16 | 0.65 | 0.54 |
| 31 | -9.54 | -8.75 | 0.79 | -- |
| 32 | -9.75 | -10.13 | 0.38 | -- |
| $R^2$ | 0.82 | | -- | -- |

**Table 6:** FEP/REST results for the 8 relative binding tree energy calculation paths of the jnk1 ligands.

| Path | dG_Exp | dG_FEP/REST |
|---|---|---|
| Fep2→6 | -0.21 | -0.72 |
| Fep2→7 | -0.18 | -0.32 |
| Fep2→9 | -0.92 | -2.47 |
| Fep2→ 10 | -0.59 | -0.46 |
| Fep6→13 | 1.41 | 0.20 |
| Fep7→ 13 | 1.38 | -0.04 |

(continued)

| Path | dG_Exp | dG_FEP/REST |
|---|---|---|
| Fep9→ 12 | -0.58 | -0.88 |
| Fep10→12 | -0.91 | -2.59 |

**Table 7:** The predicted binding affinities and their associated errors for the 7 ligands binding to jnk1.

| Ligand | dG_Exp | dG_CC_pred | CC Error |
|---|---|---|---|
| L2 | -8.49 | -8.49 | 0.0 |
| L6 | -8.70 | -9.17 | 0.1 |
| L7 | -8.67 | -8.85 | 0.1 |
| L9 | -9.41 | -10.89 | 0.2 |
| L10 | -9.08 | -9.03 | 0.2 |
| L12 | -9.99 | -11.69 | 0.2 |
| L13 | -7.29 | -8.93 | 0.1 |

**Table 8:** FEP/REST results for the 7 relative binding free energy calculation paths for the BACE ligands.

| Path | dG_Exp | dG_FEP/REST |
|---|---|---|
| Fep17d→4j | 0.40 | 0.55 |
| Fep17h→4p | 0.25 | 1.15 |
| Fep17g→4o | 0.36 | 0.63 |
| Fep17d→17h | -0.91 | -2.50 |
| Fep17d→17g | -0.32 | 0.98 |
| Fep4j→4p | -1.06 | -2.30 |
| Fep4j→4o | -0.36 | -1.00 |

**Table 9:** The predicted binding affinities and their associated errors for the 6 ligands binding to BACE.

| Ligand | dG_Exp | dG_CC_pred | CC Error |
|---|---|---|---|
| 4j | -9.02 | -9.02 | 0.0 |
| 4p | -10.08 | -11.22 | 0.2 |
| 4o | -9.38 | -10.02 | 0.2 |
| 17d | -9.42 | -9.67 | 0.2 |
| 17h | -10.33 | -12.27 | 0.2 |
| 17g | -9.74 | -10.65 | 0.2 |

**Table 10:** MC/FEP results for the 9 relative binding free energy calculation paths of scytalone dehydratase ligands.

| Path | dG_Exp | dG_MC/FEP |
|---|---|---|
| L1(H2O)→L1 | -- | 5.5 |

(continued)

| Path | dG_Exp | dG_MC/FEP |
|---|---|---|
| L2(H2O)→L2 | -- | -9.8 |
| L3(H2O)→L3 | -- | -0.4 |
| L1(H2O)→L3(H2O) | 3.8 | 6.2 |
| L1(H2O)→L2(H2O) | -2.0 | 14.1 |
| L3(H2O)→L2(H2O) | -5.8 | 3.6 |
| L1→L3 | 3.8 | 0.5 |
| L1→L2 | -2.0 | -6.3 |
| L3→L2 | -5.8 | -7.3 |

**Table 11:** The predicted binding affinities and their associated errors for the 3 ligands binding to scytalone dehydratase. Even though the predicted binding affinity for ligand 3 has relative large error, the relative binding free energy calculation paths with very large errors are filtered out and the correct rank order is predicted.

| Ligand | dG_Exp | dG_CC_pred* | CC Error |
|---|---|---|---|
| L1 | 0 | 0 | 0 |
| L2 | -1.14 | -2.0 | 0.3 |
| L3 | 5.82 | 3.8 | 0.3 |
| *Note: the binding affinities reported in the table is the relative binding affinity compared to ligand 1. | | | |

**Table 12:** The cycle closure predicted free energy differences among the four states depicted on figure 14, and their associated errors. M denotes the united atom methane, P denotes the hydrophobic plates, CP denotes the charged plates, MP denotes the hydrophobic plates bound with the united atom methane, and MCP denotes the charged plates bound with the united atom methane.

| Mutaion | dG_CC_pred | CC Error |
|---|---|---|
| M+P→MP | -2.846 | 0.04 |
| M+CP→MCP | 16.540 | 0.04 |
| P→CP | -131.004 | 0.04 |
| MP→MCP | -111.618 | 0.04 |

REFERENCES

(1) Gilson, M. K.; Zhou, H. X. Annu. Rev. Biophys. Biomol. Struct. 2007, 36, 21.
(2) Jorgensen, W. L. Acc. Chem. Res. 2009, 42, 733.
(3) Moble, D. L.; Dill, K. A. Structure 2009, 17, 498.
(4) Gallicchio, E.; Levy, R. M. Curr. Opin. Struct. Biol. 2011, 21, 161.
(5) Mobley, D. L.; Dill, K. A.; Chodera, J. D. J. Phys. Chem. B 2008, 112, 938.
(6) Young, T.; Abel, R.; Kim, B.; Berne, B. J.; Friesner, R. A. Proc. Nat. Acad. Sci. USA 2007, 104, 813.
(7) Abel, R.; Young, T.; Farid, R.; Berne, B. J.; Friesner, R. A. J. Am. Chem. Soc. 2008, 130, 2831.
(8) Wang, L.; Berne, B. J.; Friesner, R. A. Proc. Nat. Acad. Sci. USA 2011, 108, 1326.
(9) de Amorim, H. L.; Caceres, R. A.; Netz, P. A. Curr. Drug Targets 2008, 9, 1100.
(10) Michel, J.; Tirado-Rives, J.; Jorgensen, W. L. J. Am. Chem. Soc. 2009, 131, 15403.
(11) Chodera, J. D.; Mobley, D. L.; Shirts, M. R.; Dixon, R. W.; Branson, K.; Pande, V. S. Curr. Opin. Struct. Biol.

2011, 21.

(12) Wang, L.; Berne, B. J.; Friesner, R. A. Proc. Nat. Acad. Sci. USA 2012, 109, 1937.

(13) Huang, X.; Hagen, M.; Kim, B.; Friesner, R. A.; Zhou, R.; Berne, B. J. J. Phys. Chem. B 2007, 111, 5405.

(14) Liu, P.; Kim, B.; Friesner, R. A.; Berne, B. J. Proc. Nat. Acad. Sci. USA 2005, 102, 13749.

(15) Wang, L.; Friesner, R. A.; Berne, B. J. J. Phys. Chem. B 2011, 115, 9431.

(16) Hardcastle, I. R.; Arris, C. E.; Bentley, J.; Boyle, F. T.; Chen, Y.; Curtin, N. J.; Endicott, J. A.; Gibson, A. E.; Golding, B. T.; Griffin, R. J.; Jewsbury, P.; Menyerol, J.; Mesguiche, V.; Newell, D. R.; Noble, M. E.; Pratt, D. J.; Wang, L. Z.; Whitfield, H. J. J. Med. Chem. 2004, 47, 3710.

(17) Malumbres, M.; Barbacid, M. Nat. Rev. Cancer 2009, 9, 153.

(18) Chen, Y. N.; Sharma, S. K.; Ramsey, T. M.; Jiang, L.; Martin, M. S.; Baker, K.; Adams, P. D.; Bair, K. W.; Kaelin, W. G., Jr. Proc. Nat. Acad. Sci. USA 1999, 96, 4325.

(19) Pohorille, A.; Jarzynski, C.; Chipot, C. J. Phys. Chem. B 2010, 114, 10253.

(20) Steiner, D.; Oostenbrink, C.; Diederich, F.; Zurcher, M.; van Gunsteren, W. F. J. Comput. Chem. 2011, 32, 1801.

(21) Lai, B.; Oostenbrink, C. Theor. Chem. Acc. 2012, 131, 1.

(22) Jiang, W.; Roux, B. J. Chem. Theory Comput. 2010, 6, 2565.

(23) Woods, C. J.; Essex, J. W.; King, M. A. J. Phys. Chem. B 2003, 107, 13703.

(24) Patriksson, A.; van der Spoel, D. Phys. Chem. Chem. Phys. 2008, 10, 2073.

(25) Beutler, T. C.; Mark, A. E.; van Schaik, R. C.; Gerber, P. R.; van Gunsteren, W. F. Chem. Phys. Lett. 1994, 222, 529.

(26) In Desmond; 3.4 ed.; Schrodinger L. L. C.: New York, NY, 2012.

(27) Bowers, K. J.; Chow, E.; Xu, H.; Dror, R. O.; Eastwood, M. P.; Gregersen, B. A.; Klepeis, J. L.; Kolossvary, I.; Moraes, M. A.; Sacerdoti, F. D.; Salmon, J. K.; Shan, Y.; Shaw, D. E. In Proceedings of the 2006 ACM/IEEE conference on Supercomputing; ACM: Tampa, Florida, 2006, p 84.

(28) Shivakumar, D.; Williams, J.; Wu, Y.; Damm, W.; Shelley, J.; Sherman, W. J. Chem. Theory Comput. 2010, 6, 1509.

(29) Jorgensen, W. L.; Tirado-Rives, J. J. Am. Chem. Soc. 1988, 110, 1657.

(30) Kaminski, G. A.; Friesner, R. A.; Tirado-Rives, J.; Jorgensen, W. L. J. Phys. Chem. B 2001, 105, 6474.

(31) Berendsen, H. J. C.; Postma, J. P. M.; van Gunsteren, W. F.; J., H.; Pullman, B., Ed.; Reidel: Dordrecht, 1981, p 331.

(32) In Maestro; 9.3 ed.; Schrodinger, L. L. C: New York, NY, 2012.

(33) In Schrodinger Suite 2012 Protein Preparation Wizard; Schrodinger L. L. C.: New York, NY, 2012.

(34) Olsson, M. H. M.; Søndergaard, C. R.; Rostkowski, M.; Jensen, J. H. J. Chem. Theory Comput. 2011, 7, 525.

(35) Bennett, C. H. J. Comput. Phys. 1976, 22, 245.

(36) Paliwal, H.; Shirts, M. R. J. Chem. Theory Comput. 2011, 7, 4115.

(37) Hahn, A. M.; Then, H. Phys. Rev. E 2009, 80, 031111.

(38) Shenfeld, D. K.; Xu, H.; Eastwood, M. P.; Dror, R. O.; Shaw, D. E. Phys. Rev. E 2009, 80, 046705.

(39) Michel, J.; Tirado-Rives, J.; Jorgensen, W. L. J. Am. Chem. Soc. 2009, 131, 15403.

(40) Wang, L.; Friesner, R. A.; Berne, B. J. J. Phys. Chem. B 2010, 114, 7294.

**Claims**

1.  A computer-implemented method of determining relative strength of binding between a receptor and individual members of a set of ligands to form complexes between individual ligand set members and the receptor, the method comprising a computer-implemented determination of multiple relative binding free energy differences for a set of three or more ligand pairs forming at least one closed cycle, and a computer implemented determination of hysteresis magnitude about each closed thermodynamic cycle, comprising:

    a) a computer implemented probabilistic determination of the free energy differences and error distributions about those free energy differences along each of the legs of the closed thermodynamic cycle that probabilistically lead to the hysteresis value observed for the closed thermodynamic cycles;
    b) a computer implemented determination of the most probable free energy difference for each leg in the closed thermodynamic cycle included in the probabilistic model determined in step a);
    c) a computer implemented determination of the most probable error associated with the most probable binding free energy difference for each pair of ligands along each leg in the closed thermodynamic cycle from the probabilistic determination in step b);
    d) a computer implemented output of values representing the relative binding free energies and errors in step c) to a computer user.

**2.** The method of claim 1 in which the step of estimating the error comprises computer-implemented analysis of binding free energy differences between ligands along legs of more than one closed thermodynamic cycle, and computer implemented determination of hysteresis magnitude about each of closed thermodynamic cycles.

**3.** The method of claim 1 in which steps c and d include determining a set of free energy values for each leg that minimizes the function

$$\sum_i \frac{(E_i - F_i)^2}{2\sigma_i^2}$$

with the constraint:

$$\sum_i F_i = 0$$

where $E_i$ calculated free energy difference for a given leg i;
$F_i$ is the theoretical free energy difference for a given leg i;
and $\sigma_i$ is the standard deviation of the calculated free energy differences for leg i, and where the sum of the theoretical free energy differences for all closed cycles is 0.

**4.** The method of claim 1, where the ligands are congeneric.

**5.** The method of claim 1, where a Gaussian distribution is assumed in the construction of the probabilistic model of the observed hysteresis in step a).

**6.** The method of claim 1, where the error distribution associated with the free energy simulations is assumed to be uniform in step a).

**7.** The method of claim 1, where the error distribution associated with the free energy simulations is assumed to be additive with the Bennett error in step a).

**8.** The method of claim 1, where the connectivity of the closed thermodynamic cycles is represented as a graph.

**9.** The method of claim 1, where the connectivity of the closed thermodynamic cycles is represented as a matrix.

**10.** The method of claim 1, where the probabilistic determination comprises performing graph theoretical methods.

**11.** The method of claim 1, where the probabilistic determination comprises performing matrix algebra methods.

**12.** The method of claim 1, where the probabilistic determination comprises performing Bayesian methods.

**13.** The method of claim 1, where the probabilistic determination comprises performing Maximum likelihood methods.

**14.** A computer-readable storage medium comprising tangible non-transitory instructions stored thereon that are executable by a processing device, and upon such execution cause the processing device to perform operations according to any one of claims 1 to 13.

**15.** A computer system programmed with non-transitory computer readable instructions for performing operations according to any one of claims 1 to 13.

**Patentansprüche**

**1.** Computerimplementiertes Verfahren zum Bestimmen der relativen Bindungsstärke zwischen einem Rezeptor und individuellen Mitgliedern eines Satzes von Liganden, um Komplexe zwischen individuellen Ligandensatzmitgliedern und dem Rezeptor zu bilden, wobei das Verfahren eine computerimplementierte Bestimmung von mehreren relativen Differenzen der freien Energie der Bindung für einen Satz von drei oder mehr Ligandenpaaren, die mindestens einen geschlossenen Kreisprozess bilden, und eine computerimplementierte Bestimmung des Hysteresebetrags über jeden geschlossenen thermodynamischen Kreisprozess umfasst, umfassend:

a) eine computerimplementierte probabilistische Bestimmung der Differenzen der freien Energie und Fehlerverteilungen über jene Differenzen der freien Energie entlang jedem der Schenkel des geschlossenen thermodynamischen Kreisprozesses, die probabilistisch zu dem Hysteresewert führen, der für die geschlossenen thermodynamischen Kreisprozesse beobachtet wird;

b) eine computerimplementierte Bestimmung der wahrscheinlichsten Differenz der freien Energie für jeden Schenkel in dem geschlossenen thermodynamischen Kreisprozess, der in das probabilistische Modell eingeschlossen ist, das in Schritt a) bestimmt wurde;

c) eine computerimplementierte Bestimmung des wahrscheinlichsten Fehlers, der mit der wahrscheinlichsten Differenz der freien Bindungsenergie für jedes Paar von Liganden entlang jedes Schenkels in dem geschlossenen thermodynamischen Kreisprozess aus der probabilistischen Bestimmung in Schritt b) assoziiert ist;

d) eine computerimplementierte Ausgabe von Werten, die die relativen freien Bindungsenergien und Fehler in Schritt c) darstellen, an einen Computerbenutzer.

2. Verfahren nach Anspruch 1, wobei der Schritt des Schätzens des Fehlers computerimplementierte Analyse von Differenzen der freien Bindungsenergie zwischen Liganden entlang Schenkeln von mehr als einem geschlossenen thermodynamischen Kreisprozess und computerimplementierte Bestimmung des Hysteresebetrags über jeden der geschlossenen thermodynamischen Kreisprozesse umfasst.

3. Verfahren nach Anspruch 1, wobei Schritte c) und d) Bestimmen eines Satzes von freien Energiewerten für jeden Schenkel einschließen, der die Funktion

$$\sum_i \frac{(E_i - F_i)^2}{2\sigma_i^2}$$

mit der Randbedingung:

$$\sum_i F_i = 0$$

minimiert, wobei $E_i$ die Differenz der berechneten freien Energie für einen gegebenen Schenkel i ist;
$F_i$ die Differenz der theoretischen freien Energie für einen gegebenen Schenkel i ist;
und $\sigma_i$ die Standardabweichung der berechneten Differenzen der freien Energie für den Schenkel i ist, und wobei die Summe der Differenzen der theoretischen freien Energie für alle geschlossenen Kreisprozesse 0 ist.

4. Verfahren nach Anspruch 1, wobei die Liganden artverwandt sind.

5. Verfahren nach Anspruch 1, wobei zum Aufbau des probabilistischen Modells der beobachteten Hysterese in Schritt a) eine Gauß-Verteilung angenommen wird.

6. Verfahren nach Anspruch 1, wobei die mit den Simulationen der freien Energie assoziierte Fehlerverteilung in Schritt a) als einheitlich angenommen wird.

7. Verfahren nach Anspruch 1, wobei die mit den Simulationen der freien Energie assoziierte Fehlerverteilung in Schritt a) als additiv mit dem Bennett-Fehler angenommen wird.

8. Verfahren nach Anspruch 1, wobei die Verschaltung der geschlossenen thermodynamischen Kreisläufe als Graph dargestellt wird.

9. Verfahren nach Anspruch 1, wobei die Verschaltung der geschlossenen thermodynamischen Kreisläufe als Matrix dargestellt wird.

**10.** Verfahren nach Anspruch 1, wobei die probabilistische Bestimmung Durchführung von graphentheoretischen Verfahren umfasst.

**11.** Verfahren nach Anspruch 1, wobei die probabilistische Bestimmung Durchführung von Matrixalgebraverfahren umfasst.

**12.** Verfahren nach Anspruch 1, wobei die probabilistische Bestimmung Durchführung von Bayes'schen Verfahren umfasst.

**13.** Verfahren nach Anspruch 1, wobei die probabilistische Bestimmung Durchführung von Maximumwahrscheinlichkeitsverfahren umfasst.

**14.** Computerlesbares Speichermedium, das dingliche nicht-flüchtige Anweisungen umfasst, die darauf gespeichert sind, die durch eine Verarbeitungsvorrichtung ausführbar sind und bei dieser Ausführung bewirken, dass die Verarbeitungsvorrichtung Operationen gemäß einem der Ansprüche 1 bis 13 durchführt.

**15.** Computersystem, das mit nicht-flüchtigen computerlesbaren Anweisungen zur Durchführung von Operationen gemäß einem der Ansprüche 1 bis 13 programmiert ist.

## Revendications

**1.** Procédé, mis en œuvre par ordinateur, de détermination de la force relative d'une liaison entre un récepteur et des membres individuels d'un ensemble de ligands pour former des complexes entre des membres individuels de l'ensemble de ligands et le récepteur, le procédé comprenant une détermination mise en œuvre par ordinateur de multiples différences d'énergie libre de liaison relative pour un ensemble d'au moins trois paires de ligands formant au moins un cycle fermé, et une détermination mise en œuvre par ordinateur de l'amplitude d'hystérésis autour de chaque cycle thermodynamique fermé, comprenant :

   a) une détermination probabiliste mise en œuvre par ordinateur des différences d'énergie libre et de distributions d'erreur autour de ces différences d'énergie libre le long de chacune des branches du cycle thermodynamique fermé qui conduisent de façon probabiliste à la valeur d'hystérésis observée pour les cycles thermodynamiques fermés ;
   b) une détermination mise en œuvre par ordinateur de la différence d'énergie libre la plus probable pour chaque branche dans le cycle thermodynamique fermé inclus dans le modèle probabiliste déterminé à l'étape a) ;
   c) une détermination mise en œuvre par ordinateur de l'erreur la plus probable associée à la différence d'énergie libre de liaison la plus probable pour chaque paire de ligands le long de chaque branche dans le cycle thermodynamique fermé issue de la détermination probabiliste à l'étape b) ;
   d) une délivrance mise en œuvre par ordinateur de valeurs représentant les énergies libres de liaison relatives et les erreurs à l'étape c) à un utilisateur de l'ordinateur.

**2.** Procédé de la revendication 1 dans lequel l'étape d'estimation de l'erreur comprend l'analyse mise en œuvre par ordinateur de différences d'énergie libre de liaison entre ligands le long de branches de plus d'un cycle thermodynamique fermé, et la détermination mise en œuvre par ordinateur de l'amplitude d'hystérésis autour de chacun des cycles thermodynamiques fermés.

**3.** Procédé de la revendication 1 dans lequel les étapes c et d comportent la détermination d'un ensemble de valeurs d'énergie libre pour chaque branche qui minimise la fonction

$$\sum_i \frac{(E_i - F_i)^2}{2\sigma_i^2}$$

avec la contrainte :

$$\sum_i F_i = 0$$

où $E_i$ est la différence d'énergie libre calculée pour une branche donnée $i$ ;
$F_i$ est la différence d'énergie libre théorique pour une branche donnée $i$ ;
et $\sigma_i$ est l'écart type des différences d'énergie libre calculées pour la branche $i$, et
où la somme des différences d'énergie libre théoriques pour tous les cycles fermés est égale à 0.

4. Procédé de la revendication 1, dans lequel les ligands sont congénères.

5. Procédé de la revendication 1, dans lequel une distribution gaussienne est supposée dans la construction du modèle probabiliste de l'hystérésis observée à l'étape a).

6. Procédé de la revendication 1, dans lequel la distribution d'erreur associée aux simulations d'énergie libre est supposée être uniforme à l'étape a).

7. Procédé de la revendication 1, dans lequel la distribution d'erreur associée aux simulations d'énergie libre est supposée être additive avec l'erreur de Bennett à l'étape a).

8. Procédé de la revendication 1, dans lequel la connectivité des cycles thermodynamiques fermés est représentée sous la forme d'un graphe.

9. Procédé de la revendication 1, dans lequel la connectivité des cycles thermodynamiques fermés est représentée sous la forme d'une matrice.

10. Procédé de la revendication 1, dans lequel la détermination probabiliste comprend la réalisation de procédés théoriques graphiques.

11. Procédé de la revendication 1, dans lequel la détermination probabiliste comprend la réalisation de procédés algébriques matriciels.

12. Procédé de la revendication 1, dans lequel la détermination probabiliste comprend la réalisation de procédés bayésiens.

13. Procédé de la revendication 1, dans lequel la détermination probabiliste comprend la réalisation de procédés de probabilité maximale.

14. Support de stockage lisible par ordinateur sur lequel sont stockées des instructions tangibles non transitoires qui sont exécutables par un dispositif de traitement, et lors d'une telle exécution, conduisent le dispositif de traitement à réaliser des opérations selon l'une quelconque des revendications 1 à 13.

15. Système informatique programmé avec des instructions non transitoires lisibles par ordinateur pour réaliser des opérations selon l'une quelconque des revendications 1 à 13.

FIG. 1

FIG. 2

FIG. 3

FIG. 4

FIG. 5

FIG. 6

FIG. 7

FIG. 8

FIG. 9

FIG. 10

FIG. 11

FIG. 12

EP 2 972 311 B1

FIG. 13

EP 2 972 311 B1

FIG. 14

Computers

110

Data stores

120

Program modules

130

## FIG. 15

List the calculated free energy for all the mutations — 302

List all the closed cycles and build up the Cycle Closure Connectivity matrix (C3M) — 304

Decompose the whole graph into independent subgraphs — 306

## FIG. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 84003913
- US 20100241411 A

**Non-patent literature cited in the description**

- **WANG et al.** *PNAS,* vol. 109, 6
- **COZZINI et al.** *Curr. Med. Chem.,* vol. 11, 10
- **BEVERIDGE et al.** *Ann. Rev. Biophys. Bioeng.,* vol. 18, 1
- **GILSON, M. K. ; ZHOU, H. X.** *Annu. Rev. Biophys. Biomol. Struct.,* 2007, vol. 36, 21
- **JORGENSEN, W. L.** *Acc. Chem. Res.,* 2009, vol. 42, 733
- **MOBLE, D. L. ; DILL, K. A.** *Structure,* 2009, vol. 17, 498
- **GALLICCHIO, E. ; LEVY, R. M.** *Curr. Opin. Struct. Biol.,* 2011, vol. 21, 161
- **MOBLEY, D. L. ; DILL, K. A. ; CHODERA, J. D.** *J. Phys. Chem. B,* 2008, vol. 112, 938
- **YOUNG, T. ; ABEL, R. ; KIM, B. ; BERNE, B. J. ; FRIESNER, R. A.** *Proc. Nat. Acad. Sci. USA,* 2007, vol. 104, 813
- **ABEL, R. ; YOUNG, T. ; FARID, R. ; BERNE, B. J. ; FRIESNER, R. A.** *J. Am. Chem. Soc.,* 2008, vol. 130, 2831
- **WANG, L. ; BERNE, B. J. ; FRIESNER, R. A.** *Proc. Nat. Acad. Sci. USA,* 2011, vol. 108, 1326
- **DE AMORIM, H. L. ; CACERES, R. A. ; NETZ, P. A.** *Curr. Drug Targets,* 2008, vol. 9, 1100
- **MICHEL, J. ; TIRADO-RIVES, J. ; JORGENSEN, W. L.** *J. Am. Chem. Soc.,* 2009, vol. 131, 15403
- **CHODERA, J. D. ; MOBLEY, D. L. ; SHIRTS, M. R. ; DIXON, R. W. ; BRANSON, K. ; PANDE, V. S.** *Curr. Opin. Struct. Biol.,* 2011, vol. 21
- **WANG, L. ; BERNE, B. J. ; FRIESNER, R. A.** *Proc. Nat. Acad. Sci. USA,* 2012, vol. 109, 1937
- **HUANG, X. ; HAGEN, M. ; KIM, B. ; FRIESNER, R. A. ; ZHOU, R. ; BERNE, B. J.** *J. Phys. Chem. B,* 2007, vol. 111, 5405
- **LIU, P. ; KIM, B. ; FRIESNER, R. A. ; BERNE, B. J.** *Proc. Nat. Acad. Sci. USA,* 2005, vol. 102, 13749
- **WANG, L. ; FRIESNER, R. A. ; BERNE, B. J.** *J. Phys. Chem. B,* 2011, vol. 115, 9431
- **HARDCASTLE, I. R. ; ARRIS, C. E. ; BENTLEY, J. ; BOYLE, F. T. ; CHEN, Y. ; CURTIN, N. J. ; ENDICOTT, J. A. ; GIBSON, A. E. ; GOLDING, B. T. ; GRIFFIN, R. J.** *J. Med. Chem.,* 2004, vol. 47, 3710
- **MALUMBRES, M. ; BARBACID, M.** *Nat. Rev. Cancer,* 2009, vol. 9, 153
- **CHEN, Y. N. ; SHARMA, S. K. ; RAMSEY, T. M. ; JIANG, L. ; MARTIN, M. S. ; BAKER, K. ; ADAMS, P. D. ; BAIR, K. W. ; KAELIN, W. G., JR.** *Proc. Nat. Acad. Sci. USA,* 1999, vol. 96, 4325
- **POHORILLE, A. ; JARZYNSKI, C. ; CHIPOT, C.** *J. Phys. Chem. B,* 2010, vol. 114, 10253
- **STEINER, D. ; OOSTENBRINK, C. ; DIEDERICH, F. ; ZURCHER, M. ; VAN GUNSTEREN, W. F.** *J. Comput. Chem.,* 2011, vol. 32, 1801
- **LAI, B. ; OOSTENBRINK, C.** *Theor. Chem. Acc.,* 2012, vol. 131, 1
- **JIANG, W. ; ROUX, B.** *J. Chem. Theory Comput.,* 2010, vol. 6, 2565
- **WOODS, C. J. ; ESSEX, J. W. ; KING, M. A.** *J. Phys. Chem. B,* 2003, vol. 107, 13703
- **PATRIKSSON, A. ; VAN DER SPOEL, D.** *Phys. Chem. Chem. Phys.,* 2008, vol. 10, 2073
- **BEUTLER, T. C. ; MARK, A. E. ; VAN SCHAIK, R. C. ; GERBER, P. R. ; VAN GUNSTEREN, W. F.** *Chem. Phys. Lett.,* 1994, vol. 222, 529
- In Desmond. Schrodinger L. L. C, 2012
- **BOWERS, K. J. ; CHOW, E.; XU, H. ; DROR, R. O. ; EASTWOOD, M. P. ; GREGERSEN, B. A. ; KLEPEIS, J. L. ; KOLOSSVARY, I. ; MORAES, M. A. ; SACERDOTI, F. D. ; SALMON, J. K.** In Proceedings of the 2006 ACM/IEEE conference on Supercomputing. ACM, 2006, 84
- **SHIVAKUMAR, D. ; WILLIAMS, J. ; WU, Y. ; DAMM, W. ; SHELLEY, J. ; SHERMAN, W.** *J. Chem. Theory Comput.,* 2010, vol. 6, 1509
- **JORGENSEN, W. L. ; TIRADO-RIVES.** *J. J. Am. Chem. Soc.,* 1988, vol. 110, 1657
- **KAMINSKI, G. A. ; FRIESNER, R. A. ; TIRADO-RIVES, J. ; JORGENSEN, W. L.** *J. Phys. Chem. B,* 2001, vol. 105, 6474
- In Maestro. Schrodinger, L. L. C, 2012
- In Schrodinger Suite 2012 Protein Preparation Wizard. Schrodinger L. L. C, 2012
- **OLSSON, M. H. M ; SØNDERGAARD, C. R. ; ROSTKOWSKI, M. ; JENSEN, J. H.** *J. Chem. Theory Comput.,* 2011, vol. 7, 525

- **BENNETT, C. H.** *J. Comput. Phys.,* 1976, vol. 22, 245
- **PALIWAL, H. ; SHIRTS, M. R.** *J. Chem. Theory Comput.,* 2011, vol. 7, 4115
- **HAHN, A. M. ; THEN, H.** *Phys. Rev. E,* 2009, vol. 80, 031111
- **SHENFELD, D. K. ; XU, H. ; EASTWOOD, M. P. ; DROR, R. O. ; SHAW, D. E.** *Phys. Rev. E,* 2009, vol. 80, 046705
- **WANG, L. ; FRIESNER, R. A. ; BERNE, B. J.** *J. Phys. Chem. B,* 2010, vol. 114, 7294